# EUROPEAN PATENT SPECIFICATION

(11) **EP 4 637 720 B1**
(45) Date of publication and mention of the grant of the patent: **18.11.2026**
(21) Application number: 23833892.5
(22) Date of filing: 22.12.2023
(51) Int. Cl.: A61K 9/14, A61K 47/10, A61K 47/38

(54) **PROCESS FOR PREPARING STABLE NANOPARTICLE AND COMPOSITION THEREOF**
VERFAHREN ZUR HERSTELLUNG STABILER NANOPARTIKEL UND ZUSAMMENSETZUNG DAVON
PROCÉDÉ DE PRÉPARATION DE NANOPARTICULE STABLE ET COMPOSITION ASSOCIÉE

(30) Priority: 22.12.2022 NL 2033807
(43) Date of publication of application: 29.10.2025
(73) Proprietor: Nanomi B.V., 7575 EJ Oldenzaal (NL)
(72) Inventor: VERWIJS, Robin, 7575 EJ Oldenzaal (NL); KOETZIER, Henrieke Leonie, 7575 EJ Oldenzaal (NL); VERBIESEN, Tim Franciscus, 7575 EJ Oldenzaal (NL); EDUARDO, Acarilia, 7575 EJ Oldenzaal (NL); FRANSSEN, Okke, 7575 EJ Oldenzaal (NL)
(74) Representative: Buzzi, Notaro & Antonielli d'Oulx S.p.A.
(86) International application number: PCT/NL2023/050687
(87) International publication number: WO 2024/136660

(56) References cited:
- WO-A2-02/055059

## Description

### FIELD OF THE INVENTION

The present invention relates to a process of preparing nanoparticles with a stable and controlled mean particle size and particle size distribution. More particularly, the nanoparticles of present invention are prepared by annealing the suspension of active pharmaceutical ingredient to a specific temperature profile, so as to allow the crystal to grow in a controlled manner with desired stable morphology and a stable final particle size distribution. Further, the present invention also relates to use of nanoparticles in the preparation of pharmaceutical composition thereof.

### BACKGROUND OF THE INVENTION

For the pharmaceutical industry drug product development resulting in increased safety and efficacy is of utmost importance. In successful formulation of a drug product variables like solubility, stability, compatibility with solvent, excipient, and photostability play a critical role. Another important intrinsic parameter is particle size as it determines to a large extend the bioavailability of the drug. Controlling these variables help obtain uniform blood level drug release and thereby greatly enhancing clinical efficacy of the product.

So far numerous newly discovered active pharmaceutical ingredients are lipophilic or poorly water-soluble compounds. Designing of such active pharmaceutical ingredients into a successful drug delivery system involves many hurdles. However, many conventional approaches are available to solve the problems of low solubility, low bioavailability and stability of drugs include micronization, use of fatty solutions, use of penetration enhancer or cosolvents, surfactant dispersion method, salt formation, precipitation, etc., but still, these techniques have limited utility in solubility enhancement for poorly soluble drugs. Additional approaches like liposomes, dispersion of solids, emulsion and microemulsion methods, and inclusion complexes with cyclodextrins, which show beneficial effect as drug delivery system but major problems of these techniques are lack of universal applicability to all drugs.

Nanotechnology, in particular manufacturing of nanoparticles offer a viable alternative due to their unique and often advantageous properties. The production of active pharmaceutical ingredient particles in the nano range enhance aqueous solubility, dissolution rate, as well as bioavailability because the smaller particle provides an increased surface area to volume ratio. Traditionally, various techniques are employed to prepare nanoparticles viz. milling, liquid antisolvent precipitation, high-pressure homogenization, sol-gel method, spray pyrolysis, combustion method, electrochemical synthesis, emulsification technology etc.

The milling process converts larger macro-scale or micro-scale particles into smaller ones by the application of mechanical energy and the resulting particles are further air classified to recover nanoparticles. Various mills are available for this purpose such as ball mill, planetary ball mill, air-jet mill, hammer mill, pin mill. This is most commonly used technique in the pharmaceutical industry for particle size reduction. However, this technique provides low efficiency due to high-energy consumption, non-uniformity in the particle size distribution, the heterogeneous particle shape etc.

EP1620193 B1 discloses a process for solidification of inorganic or organic compounds using a novel antisolvent solidification technique. In this, a liquid medium comprising at least one dissolved organic or inorganic compound are forced through a membrane, which is positioned in a membrane module, into one or more antisolvents or vice versa to get solid particles of organic and/or inorganic compound(s).

Ghaffarian, Hamid Reza et al (Iran. J. Chem. Chem. En., 30(1):1-6, 2011) discloses a method to prepare ZnO Nanoparticles. To synthesize ZnO nanoparticles, at first the precursor solution was atomized by nebulizer under the pressure of air (7 bar), to form the droplets which were decomposed inside the reactor, where the temperature was 1200ºC. The obtained nanoparticles were collected into the cold precipitator and dried in oven at 100ºC. However, instability and poor particle size distribution of nanoparticles are the major drawbacks of this technique, leading to poor physical and chemical characteristics.

Nurul Nadia Mohd Zorkiplia etal (Procedia Chemistry, 19: 626-631, 2016) discloses a process of synthesis NiO nanoparticles. **In** this process, Nickel (II) nitrate hexahydrate was first dissolved in isopropanol and PEG and stirred, then the pH of this solution was adjusted and it was gradually heated to from gel. The gel was dried at 200°C and then grounded to get nanoparticles of NiO. However, the drawback of this process is the use of large volumes of solvents and chemicals.

V. Jenning etal (J. Microencapsulation, 19 (1); 1-10, 2002) discloses preparation of retinol solid-lipid nanoparticles (SLN). **In** this process, a lipid was melted at 85°C and retinol was added to it. The hot lipid phase was dispersed in a surfactant solution and a premix was formed. The coarse premix was passed through the high pressure homogenizer to get retinol-loaded solid-lipid nanoparticles. However, maintenance of high temperature to melt lipids, cooling rate, particle aggregation, and compatibility of drug with lipids are the critical parameters for this process.

Rashid A. Khaydarov et al (J Nanopart Res, 11:1193-1200, 2009) discloses a method of synthesis of silver nanoparticles by electrochemical method. **In** this process, two polished silver plates were employed as the anode and the cathode. They were immersed in an electrochemical cell filled with distilled water. Silver nanoparticle solutions produced in this way were stored under ambient conditions in glass containers. However, by this technique metallic nanoparticle can only be prepared using electrochemical properties of metals.

Thus, to overcome the prior art drawbacks and also to fulfil the basic necessity, of solubility and stability, and for developing a successful drug delivery system it is essential to develop a more efficient process for preparing stable nanoparticles. More particularly, there is a need to develop an improved process for preparing nanoparticles that are with a stable and controlled mean particle size and particle size distribution.

The inventors of the present invention unexpectedly identified a process for preparing stable nanoparticle by annealing the suspension of active pharmaceutical ingredient to a specific temperature profile. The nanoparticles prepared using the process of the present invention were found to be stable even after a long-term storage. The inventors of the present invention further found that annealing the suspension of active pharmaceutical ingredient to a specific temperature profile has a direct impact on the resulting particle size distribution and dissolution rate, as it allows for controlled growth of the particles to precisely tune the particle size. It was also found that, the process of the present invention promotes a well-defined single crystals morphology and separates agglomerates. The inventors of the present invention found that the nanoparticles of present invention showed a significant improvement in stability compared nanoparticle formed without annealing at a specific temperature profile.

### SUMMARY OF THE INVENTION

The aspect of the present invention is to provide a process for preparing stable nanoparticles.

Another aspect of the present invention is to provide a process for preparing nanoparticles with a stable and controlled mean particle size and particle size distribution.

One another aspect of the present invention is to provide a process for preparing nanoparticles by annealing the suspension of active pharmaceutical ingredient to a specific temperature profile.

Another aspect of the present invention is to provide a process for preparing nanoparticles by annealing the suspension of active pharmaceutical ingredient to a specific temperature profile, to provide nanoparticles with mean particle size less than 30,000 nm.

Yet another aspect of the present invention is to provide a process for preparing nanoparticles by annealing the suspension of active pharmaceutical ingredient to a specific temperature profile, such that process results in production of sterile nanoparticles.

One aspect of the present invention is to provide a process for preparing stable nanoparticles, wherein said nanoparticles are prepared by a process comprising:
a. dissolving active pharmaceutical ingredient in one or more organic solvent(s) optionally containing a surfactant;
b. adding the solution of step (a) to another solvent to produce a suspension of active pharmaceutical ingredient, wherein another solvent optionally contains a surfactant;
c. removing the organic solvent(s);
d. concentrating the resulting suspension of step (c);
e. further annealing the suspension either after step (b) or step (c) or step (d) at a temperature ranging from 30°C to 99°C for 0.5 hours to 2 weeks, at rate from 0.01°C/min to 50.0°C/min.
f. optionally, drying the nanoparticles of step (e).

One aspect of the present invention is to provide a process for preparing stable nanoparticles wherein said nanoparticles are prepared by a process comprising:
a. dissolving active pharmaceutical ingredient in one or more organic solvent(s);
b. adding the solution of step (a) to another solvent to produce a suspension of active pharmaceutical ingredient;
c. removing the organic solvent(s);
d. concentrating the resulting suspension of step (c);
e. further annealing the suspension either after step (b) or step (c) or step (d) at a temperature ranging from 30°C to 40°C for 0.5 hours to 2 weeks, at rate from 0.01°C/min to 50.0°C/min.
f. optionally, drying the nanoparticles of step (e).

Another aspect of the present invention is to provide a process for preparing stable nanoparticles wherein said nanoparticles are prepared by a process comprising:
a. dissolving active pharmaceutical ingredient in one or more organic solvent(s);
b. adding the solution of step (a) to another solvent to produce a suspension of active pharmaceutical ingredient;
c. removing the organic solvent(s);
d. concentrating the resulting suspension of step (c);
e. further annealing the suspension either after step (b) or step (c) or step (d) at a temperature ranging from 45°C to 65°C for 24 hours to 1 week, at rate from 0.1°C/min to 1.0°C/min.
f. optionally, drying the nanoparticles of step (e).

Yet another aspect of the present invention is to provide a process for preparing stable nanoparticles wherein said nanoparticles are prepared by a process comprising:
a. dissolving active pharmaceutical ingredient **in** one or more organic solvent(s);
b. adding the solution of step (a) to another solvent to produce a suspension of active pharmaceutical ingredient;
c. removing the organic solvent(s);
d. concentrating the resulting suspension of step (c);
e. further annealing the suspension either after step (b) or step (c) or step (d) at a temperature ranging from 75°C to 95°C for 24 hours to 1 week at rate from 0.2°C/min to 0.8°C/min.
f. optionally, drying the nanoparticles of step (e).

One more aspect of the present invention is to provide a process for preparing stable nanoparticles wherein step (a) and/or step (b) optionally includes surfactant.

One aspect of the present invention is to provide a pharmaceutical composition comprising nanoparticles obtained by the process of the present invention.

Yet another aspect of the present invention is to provide a pharmaceutical composition comprising nanoparticles obtained by the process of the present invention, wherein the composition is selected from oral, parenteral, transdermal, rectal, urethral, intranasal, intra respiratory, intraocular, or conjunctival.

Yet another aspect of the present invention is to provide a pharmaceutical composition comprising nanoparticles obtained by the process of the present invention, and optionally comprising one or more pharmaceutically acceptable excipients.

### BRIEF DESCRIPTION OF THE DRAWINGS

Figure 1 shows the effect of annealing (at 40°C) at different time points on the particle size distribution (PSD) performed using laser diffraction technique (SALD).
Figure 2 shows the SEM images at x2000 and x4000 magnification of the non-annealed and annealed morphology of nanoparticles produced in example 3. Figures 2A and 2C represent the non-annealed and annealed morphologies, respectively, at x2000 magnification and figures 2B and 2D represent the non-annealed and annealed morphologies, respectively, at x4000 magnification.
Figure 3 shows the release profile of a non-annealed paliperidone palmitate composition in accelerated (40 °C/ 75% RH) conditions
Figure 4 shows the release profile of a non-annealed paliperidone palmitate composition in standard (25 °C / 60% RH) conditions
Figure 5 shows the release profile of an annealed paliperidone palmitate composition at accelerated (40 °C/ 75% RH) conditions
Figure 6 shows the release profile of an annealed paliperidone palmitate composition at standard (25 °C / 60% RH) conditions
Figure 7 shows the effect of different annealing temperatures on the particle size distribution using disc centrifuge technique (CPS)
Figure 8 shows the release profile of an annealed aripiprazole composition at accelerated (40 °C/ 75% RH) conditions
Figure 9 shows the release profile of an annealed aripiprazole composition at standard (25 °C / 60% RH) conditions

### DETAILED DESCRIPTION

The present invention relates to a process of preparing stable nanoparticles and pharmaceutical composition thereof.

The invention relates to a process of preparing stable nanoparticles with a stable and controlled mean particle size and particle size distribution.

Particularly the invention relates to a process for preparing nanoparticles by applying a specific temperature profile to the suspension of active pharmaceutical ingredient. The process of the present invention involving temperature treatment promotes the nanoparticles with a well-defined single crystals morphology and separates agglomerates.

More particularly, the invention relates to a process for preparing stable nanoparticles, wherein said nanoparticles are prepared by a process comprising:
a. dissolving active pharmaceutical ingredient in one or more organic solvent(s) optionally containing a surfactant;
b. adding the solution of step (a) to another solvent to produce a suspension of active pharmaceutical ingredient, wherein another solvent optionally contains a surfactant;
c. removing the organic solvent(s);
d. concentrating the resulting suspension of step (c);
e. further annealing the suspension either after step (b) or step (c) or step (d) at a temperature ranging from 30°C to 99°C for 0.5 hours to 2 weeks, at rate from 0.01°C/min to 50.0°C/min.
f. optionally, drying the nanoparticles of step (e).

As used herein, the term "stable" refers to the particles obtained by the process of the invention such that there is no statistically significant change over time in the particle size or the particle size distribution. Stability is demonstrated by specific stability studies, in which critical quality attributes of the product are measured over time upon storage under pre- defined conditions which includes, but not limited to appearance of the suspension, appearance of container closure system, visible particulates in injections, assay and related substances, dissolution, and particle size distribution.

As used herein, the term "dissolution rate" refers to the rate at which the active pharmaceutical ingredient dissolves in the dissolution medium.

As used herein, the term "mean particle size" refers to the particle with identical dimensions in a total mass of the particulate system. It is weighted average volume mean diameter or surface area average of the total representative population of the particles. The particle size distribution of the present invention may be measured using laser-light scattering (LLS)methods, disc centrifuge, zeta sizer and dynamic light scattering (DLS) and mean particle size is calculated from the particle size distribution.

The term "nanoparticles" includes particles having an average size less than 30000 nm, preferably particles having an average size less than 25000 nm, 20000 nm, 15000 nm, 10000 nm or 5000 nm. Most preferably, the nanoparticles have an average size less than 5000 nm.

As used herein, the term "annealing" refers to a temperature treatment that alters the physical and sometimes chemical properties of a material. Annealing step helps the particles to obtain their final state, morphology and size.

The term "active pharmaceutical ingredient" is used in broad sense to include its pharmaceutically acceptable derivatives thereof. Suitable pharmaceutically acceptable derivatives include pharmaceutically acceptable solvates, pharmaceutically acceptable hydrates, pharmaceutically acceptable anhydrates, pharmaceutically acceptable enantiomers, pharmaceutically acceptable esters, pharmaceutically acceptable isomers, pharmaceutically acceptable polymorphs, pharmaceutically acceptable prodrugs, pharmaceutically acceptable tautomers, pharmaceutically acceptable complexes etc.

**In** the present invention, the active pharmaceutical ingredients are selected from the drugs that are used to treat disease/ disorder selected from but not limited to abdominal aortic aneurysm, acanthamoeba infection, adverse childhood experiences, acinetobacter infection, acquired immune deficiency syndrome, acute flaccid myelitis, adenovirus infection, attention deficit/hyperactivity disorder, atrial fibrillation, african trypanosomiasis, alkhurma hemorrhagic fever, amyotrophic lateral sclerosis, intestinal amebiasis, american trypanosomiasis, amyotrophic lateral sclerosis, anaplasmosis, ancylostoma duodenale infection, necator americanus infection, angiostrongylus infection, anisakiasis, anthrax, arenavirus infections, childhood arthritis, fibromyalgia, gout, ascaris infection, aseptic meningitis, aspergillosis, asthma, autism, avian influenza, burkholderia cepacia infection, babesiosis, bacterial vaginosis, balamuthia mandrillaris infection, balantidiasis, bartonella bacilliformis infection (carrión's disease), bartonella henselae infection (cat scratch disease), bartonella quintana infection (trench fever), baylisascaris infection (raccoon roundworm infection), bilharzia (schistosomiasis), bioterrorism diseases, black lung (coal workers' pneumoconioses), blastocystis hominis infection, blastomycosis, blood clotting disorders, body lice (pediculus humanus corporis), borrelia burgdorferi infection (lyme disease), louse-borne borreliosis, tick-borne borreliosis, botulism, bovine spongiform encephalopathy, brainerd diarrhea, bronchiolitis, bronchitis, brucellosis, bunyavirus infections, burkholderia cepacia infection, burkholderia mallei (glanders), burkholderia pseudomallei infection (melioidosis), herpes B virus infection, candida auris infection, clostridium difficile infection, clostridium perfringens infection, clostridium tetani infection (tetanus), cryptococcosis gattii cryptococcosis, cryptococcosis neoformans cryptococcosis, campylobacteriosis, cancer (colorectal, cervical, lung, prostate, skin, breast, esophageal, endometrial, pancreatic, hepatic, etc.), candida auris infection, canine flu, capillariasis, carbapenem-resistant enterobacterales, carbapenem resistant klebsiella pneumonia, carpal tunnel syndrome, cat flea tapeworm, crimean-congo hemorrhagic fever, cercarial dermatitis, cerebral palsy, chagas disease (trypanosoma cruzi infection), chapare hemorrhagic fever, chickenpox (varicella disease), chikungunya fever, childhood overweight and obesity, chlamydia pneumoniae infection, chlamydia psittaci infection, chlamydia trachomatis disease, vibrio cholerae infection, chronic fatigue syndrome, chronic obstructive pulmonary disease, chronic traumatic encephalopathy, chronic wasting disease, ciguatera fish poisoning, classic creutzfeldt-jakob disease, clonorchiasis, cytomegalovirus infection, coccidioidomycosis, common cold, colorado tick fever, concussion (traumatic brain injury), congenital hearing loss, congenital heart defects, conjunctivitis (pink eye), cooley's anemia, coronavirus disease 2019, corynebacterium diphtheriae infection (diphtheria), coxiella burnetii infection (Q fever), crimean-congo hemorrhagic fever, cronobacter infection, cryptosporidiosis, cryptosporidium infection (cryptosporidiosis), cyanobacterial algal bloom-associated illness, cyclosporiasis, cysticercosis, cystoisosporiasis, deep vein thrombosis, dementia, dengue, dermatophyte infection, developmental disabilities, diabetes, dientamoeba fragilis infection, diphyllobothriasis, dipylidium infection, dirofilariasis (dog heartworm), down syndrome (trisomy 21), dracunculiasis, dwarf tapeworm (hymenolepis infection), escherichia coli infection, ear infection (otitis media), eastern equine encephalitis, ebola virus disease, epstein-barr virus infection, echinococcosis, human ehrlichiosis, elephantiasis (lymphatic filariasis), elizabethkingia infection, fungal endophthalmitis, endometriosis, entamoeba histolytica infection, enterovirus D68, non-polio enterovirus infections, epidemic typhus, epilepsy, esophageal candidiasis (thrush), exserohilum rostratum (other pathogenic fungi), extensively drug-resistant TB, necrotizing fasciitis, fascioliasis, fasciolopsiasis, fetal alcohol spectrum disorders, fibromyalgia, fifth disease [parvovirus B19 infection], filovirus infections, flavivirus infections, folliculitis (hot tub rash), fragile X syndrome, francisella tularensis infection (tularemia), fungal meningitis, granulomatous amebic encephalitis (acanthamoeba), group a strep infection, group B strep infection, vulvovaginal candidiasis, genital herpes [herpes simplex virus infection], human papillomavirus infection, german measles (rubella virus), giardiasis, gnathostomiasis, gonorrhea (neisseria gonorrhoeae infection), gout, guillain-barré syndrome, guinea worm disease (dracunculiasis), H3N2V influenza, harmful algal bloom associated illness, haemophilus influenzae serotype B, hand foot and mouth disease, hansen's disease, hantavirus pulmonary syndrome, head lice (pediculus humanus capitis), heartland virus infection, hemoglobinopathies, hemophilia, viral hemorrhagic fevers, hendra virus disease, viral hepatitis, hepatitis a, hepatitis b, herpes zoster, heterophyes infection (heterophyiasis), histoplasma capsulatum infection histoplasmosis), human hookworm infestation, zoonotic hookworm infestation, human parainfluenza viruses, inflammatory bowel disease, impetigo, infectious mononucleosis, infertility, H1N1 flu, intestinal amebae infection, nonpathogenic (harmless) intestinal protozoa infection, invasive candidiasis, isospora infection (cystoisospora infection), jamestown canyon virus infection, japanese encephalitis, jaundice, klebsiella pneumonia infection, kala-azar (leishmania infection), kawasaki disease, acanthamoeba keratitis, fungal keratitis, kernicterus, kyasanur forest disease, chronic kidney disease, la crosse encephalitis, lassa fever, louse-borne relapsing fever, lymphocytic choriomeningitis, legionellosis, leishmaniasis, leprosy (hansen's disease), leptospirosis, listeriosis, loa loa infection (loiasis), lou gehrig's disease, lujo hemorrhagic fever, systemic lupus erythematosus, lymphocytic choriomeningitis, mycobacterium avium complex, malaria, marburg hemorrhagic fever, marine toxins, muscular dystrophy, multidrug-resistant TB, myalgic encephalomyelitis/ chronic fatigue syndrome, measles, melioidosis (burkholderia pseudomallei infection), middle east respiratory syndrome coronavirus, methicillin resistant staphylococcus aureus, microcephaly, microsporidia infection, molluscum contagiosum, monkey b virus infection, monkeypox, mucormycosis, mumps, mycobacterium abscessus infection, myelomeningocele, myiasis, primary amebic meningoencephalitis, amyotrophic lateral sclerosis, necrotizing fasciitis (group a strep infection), neglected tropical diseases, neisseria gonorrhoeae infection, neurocysticercosis, nocardia asteroides infection, non-polio enterovirus infections, osteoarthritis, omsk hemorrhagic fever, onchocerciasis (river blindness), opisthorchis infection, orf virus infection (sore mouth infection), oropharyngeal candidiasis (thrush), peripheral arterial disease, paragonimiasis, parainfluenza, parvovirus B19 infection (fifth disease), pneumocystis pneumonia, pulmonary embolism, pertussis (whooping cough), pinworm infection (enterobius vermicularis infection), plague (yersinia pestis infection), pneumonia, polio infection (poliomyelitis infection), pontiac fever (legionnaires' disease), powassan virus infection, poxvirus infections, primary amebic meningoencephalitis (naegleria infection), prion diseases (transmissible spongiform encephalopathies), pseudomonas aeruginosa infection, psittacosis (chlamydia psittaci infection), psoriasis, rheumatoid arthritis, rabies, rat-bite fever (strep. moniliformis infection), recreational water illnesses, respiratory syncytial virus infection, spotted fever group rickettsia, rickettsia rickettsii infection (rocky mountain spotted fever), rift valley fever, salmonella typhi infection (typhoid fever), sappinia infection, severe acute respiratory syndrome, scabies, scarlet fever (group A strep infection), schistosoma infection (schistosomiasis), trichomoniasis, sudden infant death syndrome, sinusitus, sleeping sickness (african trypanosomiasis), smallpox, southern tick-associated rash illness, sporothrix, strongyloidiasis, schizophrenia, taenia infection, thalassemia (cooley's anemia), thrombophilia, toxoplasmosis, traumatic brain injury, trichinellosis (trichinosis), trichomoniasis, trichuriasis (whipworm infection), trypanosoma cruzi infection (chagas disease), african trypanosomiasis (sleeping sickness), tourette syndrome, tularemia (francisella tularensis infection), ulcerative colitis, undulant fever (brucella infection), unexplained respiratory disease outbreaks, vaginal candidiasis, valley fever (coccidioidomycosis), vancomycin-intermediate/resistant staphylococcus aureus infections, vancomycin-resistant enterococci infection, variant creutzfeldt-jakob disease, varicella-zoster virus infection, viral hemorrhagic fevers, alkhurma hemorrhagic fever, chapare hemorrhagic fever, crimean-congo hemorrhagic fever (nairovirus infection), kyasanur forest disease, lujo hemorrhagic fever, marburg hemorrhagic fever, von willebrand disease, west nile virus infection, whipworm infection (trichuriasis), whitmore's disease (melioidosis), yeast infection, yellow fever, yersinia enterocolitica infection (yersiniosis), zika virus infection.

**In** the present invention, the organic solvent and another solvent are partially to fully miscible with each other, and the active pharmaceutical ingredients are poorly soluble in another solvent. The organic solvent according to the invention include but are not limited to tetrahydrofuran (THF), acetone, dimethyl sulfoxide, dimethylformamide, dichloromethane, acetonitrile, triethanolamine, N-Methyl-2-pyrrolidone, and acetic acid, alcohols like methanol, ethanol, propanol, butanol, alkali esters like ethyl acetate and the likes or combinations thereof.

The active pharmaceutical component(s) may be dissolved in an amount of 0.1 to 50 w/w % in the organic solvent or in the another solvent. The organic solvent and another solvent may be mixed in a ratio range from 1:2 to 1:100, preferably ratio range from 1:4 to 1:20 and more preferably ratio range 1:5 to 1:15.

The surfactant according to the invention include but are not limited to nonionic, ionic, cationic and amphoteric surfactants, such as cetyl pyridinium chloride, gelatin, casein, lecithin (phosphatides), dextran, glycerol, gum acacia, cholesterol, tragacanth, stearic acid, benzalkonium chloride, carrageenan, diethanolamine, calcium stearate, glycerol monostearate, cetostearyl alcohol, cetomacrogol emulsifying wax, sorbitan esters (Span 20, Span 80), polyoxyethylene alkyl ethers, polyoxyethylene castor oil derivatives, polyoxyethylene sorbitan fatty acid esters like polysorbate 20 or 80 (Polysorbates); ethylene glycol, glycol esters, ethylene glycol esters, polyethylene glycols, dodecyl trimethyl ammonium bromide, aliphatic alcohols, lanolin and alcohols thereof, fatty acids, mineral oils, polyoxyethylene stearates, colloidal silicon dioxide, phosphates, sodium dodecyl sulfate, carboxymethylcellulose calcium, hydroxypropyl celluloses, methylcellulose, hydroxyethylcellulose, hydroxypropylmethyl- cellulose, hydroxypropylmethyl-cellulose phthalate, non-crystalline cellulose, magnesium aluminum silicate, triethanolamine, polyvinyl alcohol, 4- (1,1,3,3-tetramethylbutyl)-phenol polymer with ethylene oxide and formaldehyde (also known as tyloxapol, superione, and triton), poloxamers; poloxamines; a charged phospholipid such as dimyristoyl phosphatidyl glycerol, dioctylsulfosuccinate (DOSS), dialkylesters of sodium sulfosuccinic acid, sodium lauryl sulfate; alkyl aryl polyether; p-isononylphenoxypoly- (glycidol),; decanoyl-N-methylglucamide; n-decyl β-D-glucopyranoside; n- decyl β-D-maltopyranoside; n-dodecyl β-D-glucopyranoside; n-dodecyl β-D- maltoside; heptanoyl-N-methylglucamide; n-heptyl-β-D-glucopyranoside; n- heptyl β-D-thioglucoside; n-hexyl β-D-glucopyranoside; nonanoyl-N-methylglucamide; n-nonyl β-D-glucopyranoside; octanoyl-N- methylglucamide; n-octyl-β-D-glucopyranoside; octyl β-D- thioglucopyranoside and the like or combinations thereof. Preferably, the surfactant used according to the present invention is selected from polysorbate 20 or 80, span 20 or 80, SDS and Poloxamers.

The surfactant component(s) may be present in either the organic solvent, another solvent or both.

The surfactant component(s) may be present in an amount of up to 15 w/w %, preferably below 4 w/w %, more preferably below 2.5 w/w % of the organic solvent or another solvent.

In the process of present invention, the organic solvent is removed using a method selected from purging with gas, distillation, extraction or supercritical fluid extraction.

In the process of present invention, the suspension is concentrated using method selected from tangential flow filtration, centrifugation/sedimentation or dead-end filtration.

**In** an embodiment, the invention relates to a process for preparing stable nanoparticles, wherein said nanoparticles are prepared by a process comprising:
a. dissolving active pharmaceutical ingredient **in** one or more organic solvent(s) optionally containing a surfactant;
b. adding the solution of step (a) to another solvent to produce a suspension of active pharmaceutical ingredient, wherein another solvent optionally contains a surfactant;
c. removing the organic solvent(s);
d. concentrating the resulting suspension of step (c);
e. further annealing the suspension either after step (b) or step (c) or step (d) at a temperature ranging from 30°C to 99°C for 0.5 hours to 2 weeks, at rate from 0.01°C/min to 50.0°C/min.
f. optionally, drying the nanoparticles of step (e).

**In** another embodiment, the invention relates to a process for preparing stable nanoparticles wherein said nanoparticles are prepared by a process comprising:
a. dissolving active pharmaceutical ingredient **in** one or more organic solvent(s) optionally containing a surfactant;
b. adding the solution of step (a) to another solvent to produce a suspension of active pharmaceutical ingredient, wherein another solvent optionally contains a surfactant;
c. removing the organic solvent(s);
d. concentrating the resulting suspension of step (c);
e. further annealing the suspension either after step (b) or step (c) or step (d) at a temperature ranging from 30°C to 40°C for 0.5 hours to 2 weeks, at rate from 0.01°C/min to 50.0°C/min.
f. optionally, drying the nanoparticles of step (e).

**In** an embodiment, the invention relates to a process for preparing stable nanoparticles wherein said nanoparticles are prepared by a process comprising:
a. dissolving active pharmaceutical ingredient in one or more organic solvent(s) optionally containing a surfactant;
b. adding the solution of step (a) to another solvent to produce a suspension of active pharmaceutical ingredient, wherein another solvent optionally contains a surfactant;
c. removing the organic solvent(s);
d. concentrating the resulting suspension of step (c);
e. further annealing the suspension either after step (b) or step (c) or step (d) at a temperature ranging from 45°C to 65°C for 24 hours to 1 week at rate from 0.1°C/min to 1.0°C/min.
f. optionally, drying the nanoparticles of step (e).

In a preferred embodiment, the invention provides a process for preparing stable nanoparticles wherein said nanoparticles are prepared by a process comprising:
a. dissolving active pharmaceutical ingredient in one or more organic solvent(s) selected from tetrahydrofuran (THF), acetone, dimethyl sulfoxide, dimethylformamide, dichloromethane, acetonitrile, triethanolamine, N-Methyl-2-pyrrolidone, and acetic acid, alcohols like methanol, ethanol, propanol, butanol, or alkali esters like ethyl acetate optionally containing a surfactant;
b. adding the solution of step (a) to another solvent selected from water, water for injection, acetone, or alcohols like methanol, ethanol, propanol, butanol to produce a suspension of active pharmaceutical ingredient, wherein another solvent optionally contains one or more surfactant;
c. removing the organic solvent(s) by purging with gas;
d. concentrating the resulting suspension of step (c) by tangential flow filtration.
e. further annealing the suspension either after step (b) or step (c) or step (d) at a temperature ranging from 55°C for 48 hours, at rate of 0.8°C/min.
f. optionally, drying the nanoparticles of step (e).

In a preferred embodiment, the invention provides a process for preparing stable nanoparticles wherein said nanoparticles are prepared by a process comprising:
a. dissolving active pharmaceutical ingredient in one or more organic solvent(s) selected from tetrahydrofuran (THF), acetone, dimethyl sulfoxide, dimethylformamide, dichloromethane, acetonitrile, triethanolamine, N-Methyl-2-pyrrolidone, and acetic acid, alcohols like methanol, ethanol, propanol, butanol, or alkali esters like ethyl acetate optionally containing a surfactant;
b. adding the solution of step (a) to another solvent selected from water, water for injection, acetone, or alcohols like methanol, ethanol, propanol, butanol to produce a suspension of active pharmaceutical ingredient, wherein another solvent optionally contains one or more surfactant;
c. removing the organic solvent(s) by purging with gas;
d. concentrating the resulting suspension of step (c) by tangential flow filtration.
e. further annealing the suspension either after step (b) or step (c) or step (d) at a temperature ranging from 65°C for 48 hours, at rate of 0.8°C/min.
f. optionally, drying the nanoparticles of step (e).

In an embodiment, the invention relates to a process for preparing stable nanoparticles wherein said nanoparticles are prepared by a process comprising:
a. dissolving active pharmaceutical ingredient in one or more organic solvent(s) optionally containing a surfactant;
b. adding the solution of step (a) to another solvent to produce a suspension of active pharmaceutical ingredient, wherein another solvent optionally contains a surfactant;
c. removing the organic solvent(s);
d. concentrating the resulting suspension of step (c);
e. further annealing the suspension either after step (b) or step (c) or step (d) at a temperature ranging from 75°C to 95°C for 24 hours to 1 week at rate from 0.2°C/min to 0.8°C/min.
f. optionally, drying the nanoparticles of step (e).

In a preferred embodiment, the invention provides a process for preparing stable nanoparticles wherein said nanoparticles are prepared by a process comprising:
a. dissolving active pharmaceutical ingredient in one or more organic solvent(s) selected from tetrahydrofuran (THF), acetone, dimethyl sulfoxide, dimethylformamide, dichloromethane, acetonitrile, triethanolamine, N-Methyl-2-pyrrolidone, and acetic acid, alcohols like methanol, ethanol, propanol, butanol, or alkali esters like ethyl acetate optionally containing a surfactant;
b. adding the solution of step (a) to another solvent selected from water, water for injection, acetone, or alcohols like methanol, ethanol, propanol, butanol to produce a suspension of active pharmaceutical ingredient, wherein another solvent optionally contains one or more surfactant;
c. removing the organic solvent(s) by purging with gas;
d. concentrating the resulting suspension of step (c) by tangential flow filtration.
e. further annealing the suspension either after step (b) or step (c) or step (d) at a temperature ranging from 80°C for 24 hours to 1 week, at rate of 0.2°C/min to 0.8°C/min.
f. optionally, drying the nanoparticles of step (e).

**In** a preferred embodiment, the invention provides a process for preparing stable nanoparticles wherein said nanoparticles are prepared by a process comprising:
a. dissolving active pharmaceutical ingredient in one or more organic solvent(s) selected from tetrahydrofuran (THF), acetone, dimethyl sulfoxide, dimethylformamide, dichloromethane, acetonitrile, triethanolamine, N-Methyl-2-pyrrolidone, and acetic acid, alcohols like methanol, ethanol, propanol, butanol, or alkali esters like ethyl acetate and one or more surfactant;
b. adding the solution of step (a) to another solvent selected from water, water for injection, acetone, or alcohols like methanol, ethanol, propanol, butanol to produce a suspension of active pharmaceutical ingredient, wherein another solvent contains one or more surfactant;
c. removing the organic solvent(s) by purging with gas;
d. concentrating the resulting suspension of step (c) by tangential flow filtration.
e. further annealing the suspension either after step (b) or step (c) or step (d) at a temperature ranging from 40°C to 80°C for 24 hours to 72 hours, at rate from 0.3°C/min to 1.0°C/min;
f. optionally, drying the nanoparticles of step (e).

The annealing step in the above embodiments of the present invention is an essential step to obtain nanoparticles with a stable and desired particle size distribution and a stable particle morphology. The annealing treatment allows for controlled growth of the particles to precisely tune the particle size and therefore physicochemical parameter of an active pharmaceutical ingredient. It also promotes a well-defined single crystals morphology and separates agglomerates. The desired particle size of the present invention is achieved by exposing the active pharmaceutical ingredient at a temperature range of a 30°C to 99°C for a specified period of time from about 0.5 hours to 2 weeks.

The particle size of the active pharmaceutical ingredient as manufactured by the present invention, also depends on the heating and cooling rate that ranges from 0.01°C/min to 50.0°C/min, preferably 0.1°C/min to 5°C/min and more preferably 0.3°C/min to 1°C/min.

According to the present invention, the size of the nanoparticles obtained is less than 30000 nm, preferably particles having an average size less than 25000 nm, 20000 nm, 15000 nm, 10000 nm or 5000 nm. Most preferably, the nanoparticles have an average size less than 5000 nm.

Another embodiment of the present invention relates to pharmaceutical composition comprising nanoparticles manufactured using the process of present invention, wherein the composition is selected from but not limited to oral, parenteral, transdermal, rectal, urethral, intranasal, intra respiratory, intraocular, or conjunctival.

More particularly, the stable nanoparticles of the present invention further are used to prepare pharmaceutical compositions including but not limited to chewable tablets, coated tablets, effervescent tablets, gastro-resistant tablets, orodispersible tablets, modified release tablets, buccal tablets, floating tablets, hard capsules, soft capsules, gastro-resistant capsules, chewable capsules, modified release capsules, effervescent powder, effervescent granules, gastro-resistant granules, lozenges, gels, pastes, pastille, cream, ointment, poultice, lotion, sponges, foam, patches, suspension, emulsion, solution, syrup, gargle, mouth wash, spray, eye drops, implants, pessaries, suppositories, parenteral suspension, parenteral emulsion, parenteral solution.

One embodiment of the present invention, the nanoparticles manufactured by the process of the invention may be used to manufacture pharmaceutical compositions that is parenteral or injection preparation in form of solution or suspension, wherein the route of administration may be intravenous, intramuscular or subcutaneous, or any other as per the requirement of the treatment.

In another embodiment of the present invention the solution or suspension for parenteral administration is prepared by diluting the resulting nanoparticles with pharmaceutically acceptable excipients; wherein the process does not involve isolation and drying the precipitated nanoparticles or freeze drying.

Moreover, the excipients used in preparation of parenteral composition of present invention include but are not limited to solubility enhancer(s), buffering agent(s), tonicity adjusting agent(s), viscosity modifying agent(s), preservative(s), bulking agent(s), wetting agent(s), pH adjusting agent(s) or vehicle(s).

The buffering agent according to the invention include but are not limited to phosphate, tartarate, ascorbate, acetate, succinate, citrate, borate, lactate, sodium phosphate, potassium phosphate, sodium dihydrogen phosphate monohydrate and the like or combinations thereof.

The tonicity adjusting agent according to the invention include but are not limited to dextrose, glycerin, mannitol, sorbitol, sodium chloride and the like or combinations thereof.

The viscosity enhancing agent according to the invention include but are not limited to methylcellulose, hydroxyethylcellulose, hydroxypropylcellulose, sodium carboxymethyl cellulose, polyvinylpyrrolidone, sodium alginate and the like or combinations thereof.

The preservative according to the invention include but are not limited to benzoic acid, benzyl alcohol, butylated hydroxyanisole, butylated hydroxytoluene, chlorbutol, a gallate, a hydroxybenzoate, EDTA, phenol, chlorocresol, metacresol, benzethonium chloride, myristyl-gamma-piccolinium chloride, phenylmercuric acetate and thimerosal. Isotonizing agents are, for example, sodium chloride, dextrose, mannitol, sorbitol, lactose, sodium sulfate. Examples of antioxidants that may also be present include, but are not limited to, acetone sodium bisulfate, ascorbate, a-tocopherol, bisulfate sodium, butylated hydroxy anisole, butylated hydroxy toluene, cystein, cysteinate HCl, dithionite sodium, gentisic acid, gentisic acid athanolamine, glutamate monosodium, formaldehyde sulfoxylate sodium, metabisulfite potassium, metabisulfite sodium, monothioglycerol, propyl gallate, sulfite sodium, tocopherol alpha, thioglycolate sodium and mixtures thereof.

The bulking agent according to the invention include but are not limited to mannitol, sorbitol, maltose, sucrose, xylitol, glucose, starches, and the like or combinations thereof.

The wetting agent according to the invention include but are not limited to glycerin, alcohol, propylene glycol, polyethylene glycol castor oil, polyethylene glycol, polyoxyethylene sorbitan fatty acid esters (polysorbates), povidone and the like or combinations thereof.

The pH adjusting agent according to the invention include but are not limited to hydrochloric acid, fumaric acid, acetic acid, citric acid, malic acid, phosphoric acid, ammonia solution, ammonium carbonate, diethanolamine, potassium hydroxide, sodium bicarbonate, sodium borate, sodium carbonate, sodium hydroxide, trolamine and the like or combinations thereof.

The following examples illustrate specific aspects and embodiments of the invention and demonstrate the practice and advantages thereof. It is to be understood that the examples are given by way of illustration only and are not intended to limit the scope of the invention in any manner.

### EXAMPLES

### Example 1: Process for preparation of the particles of Paliperidone palmitate

a. Paliperidone palmitate (54 g) was weighed in a 1 L glass Duran^{®} bottle. 846 g of Tetrahydrofuran was added to the Duran^{®} bottle to create a 6% w/w solvent phase. The mixture was then stirred until complete dissolution at room temperature.
b. 0.09 % w/w of Tween^{®} 20 (6.75 g) in water for injection (7493.25 g) was weighed in an 10L extraction vessel and cooled down to 5°C.
c. Suspension was prepared by transferring step (a) from a pressurized vessel through a flowmeter at a constant flowrate of 33mL/min, into step (b) until a ratio of water for injection to tetrahydrofuran ratio of 10:1 was reached.
d. Solvent from step (c) was extracted by introducing nitrogen into the extraction vessel while heating up back to 21°C at 20 L/min for 72h, gaseous solvent by removed from the headspace of the extraction vessel and transferred to active carbon vessels.
e. The suspension of step (d) was concentrated as by using tangential flow filtration to obtain the final concentrated suspension. Concentration was stopped when the desired paliperidone palmitate concentration of 175 mg/g was reached.
f. Annealing on step (e) was started using a Julabo heater cooler system, at 40°C for 16 days at heating and cooling rates of 0.8°C/min.

The nanoparticles produced in example 1 were characterized for their particle size distribution (PSD) using Laser Diffraction (Shimatzu-SALD-2300).

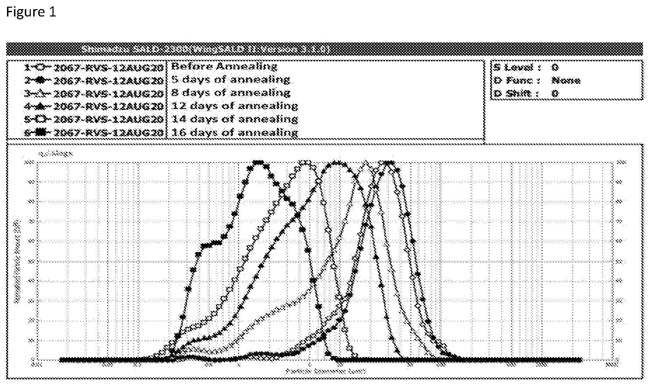

The PSD data was collected at different time points starting from 5 days up to 16 days. Table 8 and figure 1 indicates the results of PSD in terms of volume mean, D10, D50 and D90. The statistical parameters D10, D50 and D90 respectively indicate the size below which 10%, 50% and 90% of the particles are distributed. It was observed that the mean particle size was stable or slightly increased during the first 5 days of annealing. However, surprisingly the agglomerates started to dissociate and the mean particle size rapidly decreased after 5 days.

Example 2: Process for preparation of pharmaceutical composition prepared using the particles of Paliperidone palmitate manufactured according to the example 1.

**Table 1**

| **Composition of Suspension** | **Concentration (mg/mL) in Water for injection** |
|---|---|
| Paliperidone palmitate | 156.00 |
| Polysorbate 20 | 12.00 |
| Na₂HPO₄ | 5.00 |
| NaH₂PO₄*H₂O | 2.50 |
| Polyethylene Glycol 4000 | 30.00 |
| Citric Acid | 5.00 |
| Sodium Hydroxide | 2.84 |

The paliperidone palmitate nanoparticles suspension manufactured from example 1 was diluted with a buffer that contained other excipients resulting in the final drug product bulk at target API concentration including the required excipients. The resulting bulk suspension was filled in syringes or in vials.

### Example 3: Process for preparation of the particles of Paliperidone palmitate

a. Paliperidone palmitate (54 g) was weighed in a 1 L glass Duran^{®} bottle. 846 g of Tetrahydrofuran was added to the Duran^{®} bottle to create a 6% w/w solvent phase. The mixture was then stirred until complete dissolution at room temperature.
b. 0.09 % w/w of Tween^{®} 20 (6.75 g) in water for injection (7493.25 g) was weighed in an 10L extraction vessel and cooled down to 5°C.
c. Suspension was prepared by transferring step (a) from a pressurized vessel through a flowmeter at a constant flowrate of 33mL/min, into step (b) until a ratio of water for injection to tetrahydrofuran ratio of 10:1 was reached.
d. Solvent from step (c) was extracted by introducing nitrogen into the extraction vessel while heating up back to 21°C at 20 L/min for 72h, gaseous solvent by removed from the headspace of the extraction vessel and transferred to active carbon vessels.
e. The suspension of step (d) was concentrated as by using tangential flow filtration to obtain the final concentrated suspension.
   Concentration was stopped when the desired paliperidone palmitate concentration of 175 mg/g was reached.
f. Annealing on step (e) was started using a Julabo heater cooler system, at 60°C for 48 hours at heating and cooling rates of 0.8°C/min.

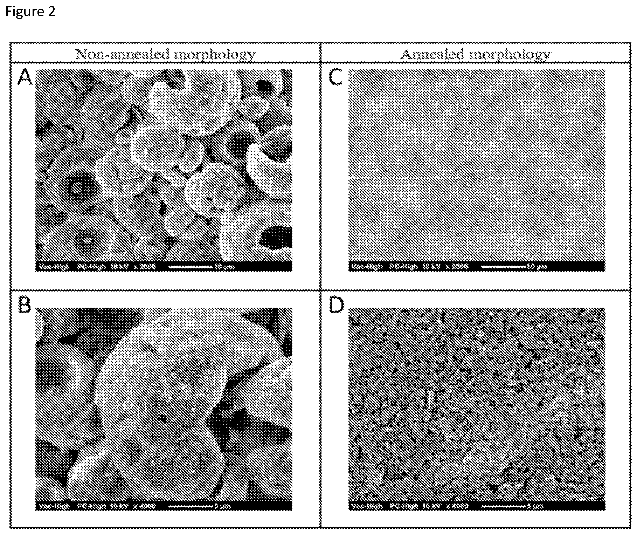

The morphologies after annealing of example 3 are captured in Scanning Electron Microscopy (SEM) images in Figure 2. The figure indicates the dissociation of agglomerates (Figure 2: A and B) after annealing treatment and resulting optimal morphology of single and separated crystals (Figure 2: C and D).

Example 4: Process for preparation of pharmaceutical composition prepared using the particles of Paliperidone palmitate manufactured according to the example 3.

**Table 2**

| **Composition of Suspension** | **Concentration (mg/mL) in Water for injection** |
|---|---|
| Paliperidone palmitate | 156.00 |
| Polysorbate 20 | 12.00 |
| Na₂HPO₄ | 5.00 |
| NaH₂PO₄*H₂O | 2.50 |
| Polyethylene Glycol 4000 | 30.00 |
| Citric Acid | 5.00 |
| Sodium Hydroxide | 2.84 |

The paliperidone palmitate nanoparticles suspension manufactured from example 3 was diluted with a buffer that contained other excipients resulting in the final drug product bulk at target API concentration including the required excipients. The resulting bulk suspension was filled in syringes or in vials.

**In** addition to the performance of the nanoparticles, their stability is also an important parameter. The nanoparticle formulations should remain stable at long-term ambient conditions and accelerated conditions. Therefore, the pharmaceutical composition manufactured from example 4 was studied at accelerated conditions at 40°C/75%RH as well as at 25°C/60%RH for long term stability. The samples were tested for various physiochemical properties such as assay, related substances, PSD, and dissolution.

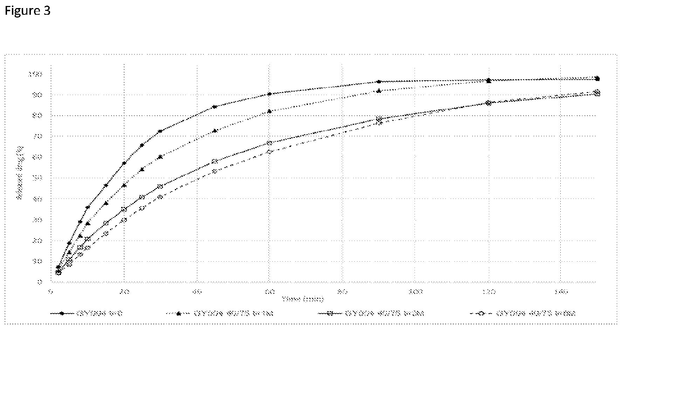

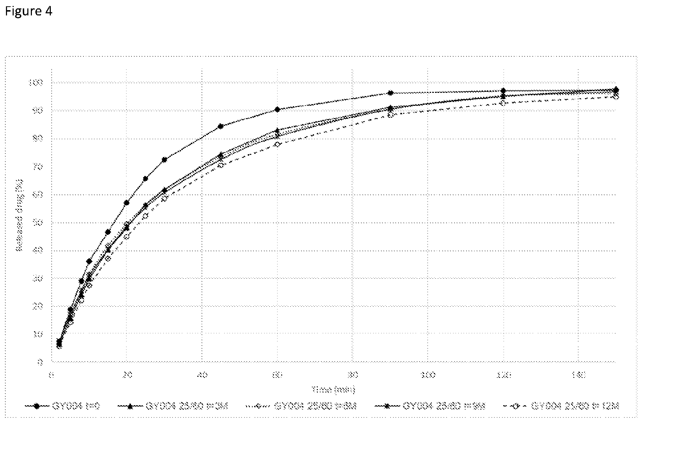

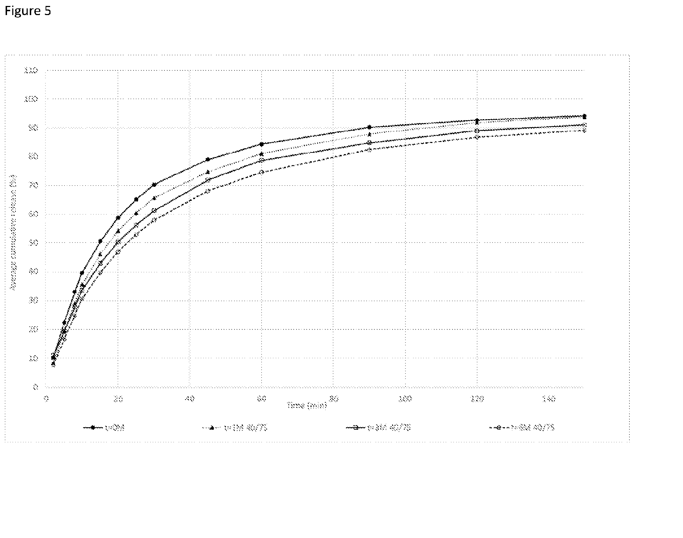

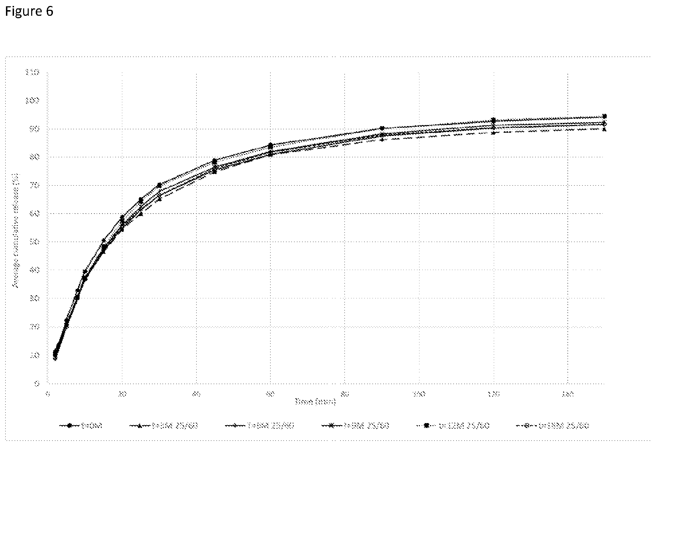

The results as reflected in table 9 and table 10 (figures 3-6) show that there was a significant increase in stability for the annealed composition in comparison to the non-annealed composition of Example 9. The decrease in dissolution rate was particularly prominent in the accelerated conditions, where the non-annealed composition showed a slower dissolution after aging. Moreover, the annealed composition showed a more stable PSD as compared to the non-annealed composition. The annealed composition showed a stability profile which reflected a more stable dissolution rate after aging, which showed a significant improvement for the product quality profile.

### Example 5: Process for preparation of the particles of Paliperidone palmitate

a. Paliperidone palmitate (54 g) was weighed in a 1 L glass Duran^{®} bottle. 846 g of Tetrahydrofuran was added to the Duran^{®} bottle to create a 6% w/w solvent phase. The mixture was then stirred until complete dissolution at room temperature.
b. 0.09 % w/w of Tween^{®} 20 (6.75 g) in water for injection (7493.25 g) was weighed in an 10L extraction vessel and cooled down to 5°C.
c. Suspension was prepared by transferring step (a) from a pressurized vessel through a flowmeter at a constant flowrate of 33mL/min, into step (b) until a ratio of water for injection to tetrahydrofuran ratio of 10:1 was reached.
d. Solvent from step (c) was extracted by introducing nitrogen into the extraction vessel while heating up back to 21°C at 20 L/min for 72h, gaseous solvent by removed from the headspace of the extraction vessel and transferred to active carbon vessels.
e. The suspension of step (d) was concentrated as by using tangential flow filtration to obtain the final concentrated suspension. Concentration was stopped when the desired paliperidone palmitate concentration of 175 mg/g was reached.
f. Annealing on step (e) was started using a Julabo heater cooler system, at 80°C for 48 hours at heating and cooling rates of 0.8°C/min.

The nanoparticles prepared in examples 1, 3 and 5 were studied for the effect of different annealing temperatures and their impact on PSD. The PSD was determined using the CPS technique. The CPS technique was used because the aggregates were dissociated, and the technique was more accurate in the capturing the particle size range of the single crystals in the range of 0.12-5 µm.

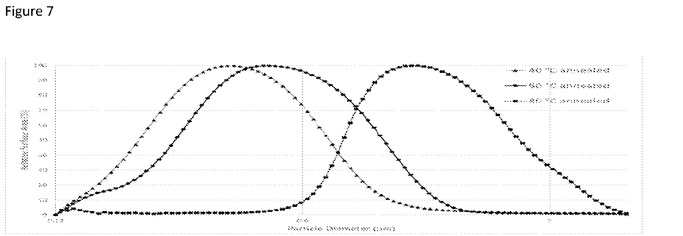

The results are summarized in Table 11 and Figure 7. During the processing, it was observed that the dissociation of aggregates occurred faster (within 24h) at higher annealing temperatures of 60 °C and 80 °C as compared to the annealing at 40 °C (5 days). The use of higher annealing temperatures resulted in a slight increase in the PSD which may allow to fine-tune the final particle size.

Example 6: Process for preparation of pharmaceutical composition prepared using the particles of Paliperidone palmitate manufactured according to the example 5.

**Table 3**

| **Composition of Suspension** | **Concentration (mg/mL) in Water for injection** |
|---|---|
| Paliperidone palmitate | 156.00 |
| Polysorbate 20 | 12.00 |
| Na₂HPO₄ | 5.00 |
| NaH₂PO₄*H₂O | 2.50 |
| Polyethylene Glycol 4000 | 30.00 |
| Citric Acid | 5.00 |
| Sodium Hydroxide | 2.84 |

The paliperidone palmitate nanoparticles suspension manufactured from example 5 was diluted with a buffer that contained other excipients resulting in the final drug product bulk at target API concentration including the required excipients. The resulting bulk suspension was filled in syringes or in vials.

### Example 7: Process for preparation of the particles of aripiprazole

a. 6 % w/w Aripiprazole (75 g) was weighed in a glass Duran^{®} bottle; 0.15 %w/w Tween^{®} 20 (1.875 g) was weighed in a small beaker. 93.85 % w/w of tetrahydrofuran (1175 g) was weighed in a beaker and used to flush Tween^{®} 20 from the beaker into the Duran^{®} bottle. The mixture was then stirred until complete dissolution at room temperature.
b. Weighed quantity of water for injection (4166.7 g) was filled in extraction vessel.
c. Suspension was prepared by transferring step (a) from a pressurized vessel through a flowmeter at a constant flowrate of 83 mL/min, into step (b) until a ratio of water for injection to tetrahydrofuran ratio of 5:1 was reached.
d. Solvent from step (c) was extracted by introducing nitrogen into the extraction vessel while heating up back to 21°C at 20 L/min for 72h, gaseous solvent by removed from the headspace of the extraction vessel and transferred to active carbon vessels.
e. The suspension of step (d) was concentrated as by using tangential flow filtration to obtain the final concentrated suspension. Concentration was stopped when the desired paliperidone palmitate concentration of 270 mg/g was reached.
f. Annealing on step (e) was started after the solvent was extracted using a Julabo heater cooler system, at 55°C for 48 hours at heating and cooling rates of 0.8°C/min.

Example 8: Process for preparation of pharmaceutical composition prepared using the particles of aripiprazole manufactured according to the example 7.

**Table 4**

| **Composition of Suspension** | **Concentration (mg/mL) in Water for injection** |
|---|---|
| Aripiprazole | 208.0 |
| Sodium Carboxymethylcellulose | 8.3 |
| Polysorbate 20 | 3.0 |
| D-Mannitol | 41.6 |
| NaH₂PO₄*H₂O | 0.7 |
| Sodium Hydroxide | 0.2 |

The aripiprazole nanoparticles suspension manufactured from example 7 was diluted with two buffers that contained other excipients results in the final drug product bulk at target API concentration including the required excipients. The resulting bulk suspension was filled in syringes or in vials.

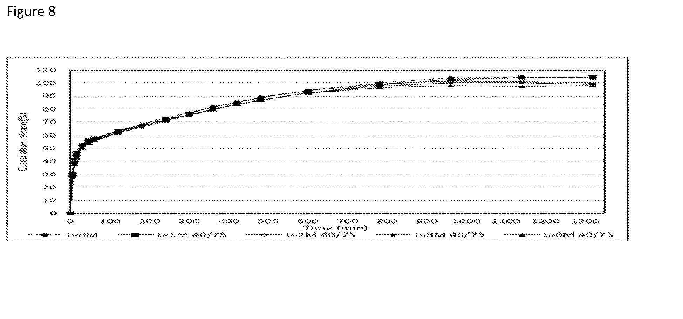

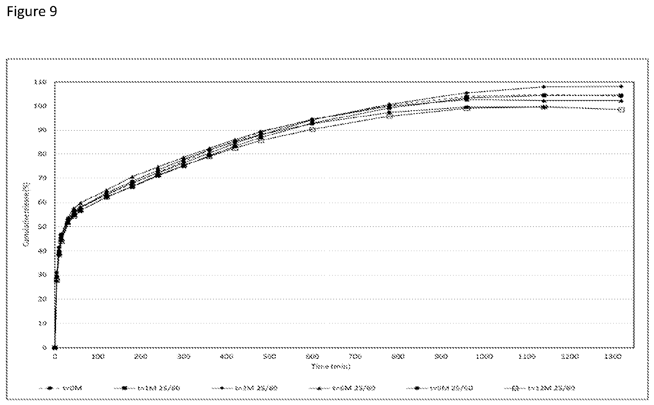

The stability of this formulation was monitored under long term stability conditions at 25°C/60%RH and accelerated conditions at 40°C/75%RH. The results are shown in Tables 12 & 13 and Figures 8 & 9 and confirm that batches made with an annealing step show significantly stable profiles.

### Example 9: Process for preparation of the particles of Paliperidone palmitate.

a. Paliperidone palmitate (54 g) was weighed in a 1 L glass Duran^{®} bottle. 846 g of Tetrahydrofuran was added to the Duran^{®} bottle to create a 6% w/w solvent phase. The mixture was then stirred until complete dissolution at room temperature.
b. 0.09 % w/w of Tween^{®} 20 (6.75 g) in water for injection (7493.25 g) was weighed in an 10L extraction vessel and cooled down to 5°C.
c. Suspension was prepared by transferring step (a) from a pressurized vessel through a flowmeter at a constant flowrate of 33mL/min, into step (b) until a ratio of water for injection to tetrahydrofuran ratio of 10:1 was reached.
d. Solvent from step (c) was extracted by introducing nitrogen into the extraction vessel while heating up back to 21°C at 20 L/min for 72h, gaseous solvent was removed from the headspace of the extraction vessel and transferred to active carbon vessels.
e. The suspension of step (d) was concentrated as by using tangential flow filtration to obtain the final concentrated suspension. Concentration was stopped when the desired paliperidone palmitate concentration of 175 mg/g was reached.

### Example 10: Process for preparation of pharmaceutical composition prepared using the particles of aripiprazole manufactured according to the example 9.

**Table 5**

| **Composition of Suspension** | **Concentration (mg/mL) in Water for injection** |
|---|---|
| Paliperidone palmitate | 156.00 |
| Polysorbate 20 | 12.00 |
| Na2HPO4 | 5.00 |
| NaH₂PO₄*H₂O | 2.50 |
| Polyethylene Glycol 4000 | 30.00 |
| Citric Acid | 5.00 |
| Sodium Hydroxide | 2.84 |

The paliperidone palmitate nanoparticles suspension manufactured from example 9 was diluted with a buffer that contained other excipients resulting in the final drug product bulk at target API concentration including the required excipients. The resulting bulk suspension was filled in syringes or in vials.

### Example 11a: Process for preparation of the particles of Paliperidone palmitate

a. Paliperidone palmitate (54 g) was weighed in a 1 L glass Duran^{®} bottle. 846 g of Tetrahydrofuran was added to the Duran^{®} bottle to create a 6% w/w solvent phase. The mixture was then stirred until complete dissolution at room temperature.
b. 0.09 % w/w of Tween^{®} 20 (6.75 g) in water for injection (7493.25 g) was weighed in an 10L extraction vessel and cooled down to 5°C.
c. Suspension was prepared by transferring step (a) from a pressurized vessel through a flowmeter at a constant flowrate of 33mL/min, into step (b) until a ratio of water for injection to tetrahydrofuran ratio of 10:1 was reached.
d. Annealing on step (c) was started using a Julabo heater cooler system, at 60°C for 48 hours at heating and cooling rates of 0.8°C/min.
e. Solvent from step (c) was extracted by introducing nitrogen into the extraction vessel while heating up back to 21°C at 20 L/min for 72h, gaseous solvent was removed from the headspace of the extraction vessel and transferred to active carbon vessels.
f. The suspension of step (e) was concentrated as by using tangential flow filtration to obtain the final concentrated suspension. Concentration was stopped when the desired paliperidone palmitate concentration of 175 mg/g was reached.

### Example 11b: Process for preparation of the particles of Paliperidone palmitate

a. Paliperidone palmitate (54 g) was weighed in a 1 L glass Duran^{®} bottle. 846 g of Tetrahydrofuran was added to the Duran^{®} bottle to create a 6% w/w solvent phase. The mixture was then stirred until complete dissolution at room temperature.
b. 0.09 % w/w of Tween^{®} 20 (6.75 g) in water for injection (7493.25 g) was weighed in an 10L extraction vessel and cooled down to 5°C.
c. Suspension was prepared by transferring step (a) from a pressurized vessel through a flowmeter at a constant flowrate of 33mL/min, into step (b) until a ratio of water for injection to tetrahydrofuran ratio of 10:1 was reached.
d. Solvent from step (c) was extracted by introducing nitrogen into the extraction vessel while heating up back to 21°C at 20 L/min for 24h, gaseous solvent was removed from the headspace of the extraction vessel and transferred to active carbon vessels.
e. Annealing on step (d) was performed using a Julabo heater cooler system, at 60°C for 48 hours at heating and cooling rates of 0.8°C/min during solvent extraction at 10L/min.
f. The suspension of step (e) was concentrated as by using tangential flow filtration to obtain the final concentrated suspension. Concentration was stopped when the desired paliperidone palmitate concentration of 175 mg/g was reached.

The impact of timing of annealing was studied in examples 11a and 11b and the results are summarized in table 14. It was observed that the presence of organic solvent impacted the growth of the particles during the annealing process. Higher solvent content in the suspension resulted in a larger average particle size using identical annealing temperature and time.

Further, when the annealing was performed earlier in the process, such as when more solvent was present in the suspension, both methods showed that PSD lead to larger particles. This moment of annealing can also be used for fine-tuning of the particle size.

Example 12a: Process for preparation of pharmaceutical composition prepared using the particles of Paliperidone palmitate manufactured according to the example 11a.

**Table 6**

| **Composition of Suspension** | **Concentration (mg/mL) in Water for injection** |
|---|---|
| Paliperidone palmitate | 156.00 |
| Polysorbate 20 | 12.00 |
| Na2HPO4 | 5.00 |
| NaH₂PO₄*H₂O | 2.50 |
| Polyethylene Glycol 4000 | 30.00 |
| Citric Acid | 5.00 |
| Sodium Hydroxide | 2.84 |

The paliperidone palmitate nanoparticles suspension manufactured from example 11a was diluted with a buffer that contained other excipients resulting in the final drug product bulk at target API concentration including the required excipients. The resulting bulk suspension was filled in syringes or in vials.

Example 12b: Process for preparation of pharmaceutical composition prepared using the particles of Paliperidone palmitate manufactured according to the example 11a.

**Table 7**

| **Composition of Suspension** | **Concentration (mg/mL) in Water for injection** |
|---|---|
| Paliperidone palmitate | 156.00 |
| Polysorbate 20 | 12.00 |
| Na2HPO4 | 5.00 |
| NaH₂PO₄*H₂O | 2.50 |
| Polyethylene Glycol 4000 | 30.00 |
| Citric Acid | 5.00 |
| Sodium Hydroxide | 2.84 |

The paliperidone palmitate nanoparticles suspension manufactured from example 11b was diluted with a buffer that contained other excipients resulting in the final drug product bulk at target API concentration including the required excipients. The resulting bulk suspension was filled in syringes or in vials.

The pharmaceutical composition manufactured from examples 12a and 12b were studied at accelerated conditions at 40°C/75%RH as well as at 25°C/60%RH for long term stability. The samples were tested for various physiochemical properties such as assay, related substances, PSD, and dissolution.

The results as reflected in table 15 showed that the annealed compositions of aripiprazole showed a satisfactory stability profile.

**Table 8: Effect of annealing at different time points**

| Annealing Duration at 40°C | SALD Analysis | | | |
|---|---|---|---|---|
| | Volume mean (µm) | D10 (µm) | D50 (µm) | D90 (µm) |
| Non-annealed | 22.04 | 8.43 | 24.36 | 50.65 |
| 5 days | 24.22 | 9.72 | 27.39 | 55.04 |
| 8 days | 9.55 | 1.87 | 12.49 | 31.20 |
| 12 days | 5.33 | 1.26 | 6.20 | 18.60 |
| 14 days | 2.47 | 0.65 | 2.88 | 7.28 |
| 16 days | 1.42 | 0.43 | 1.49 | 4.17 |

**Table 9: Stability profile of non-annealed (Example 9) and annealed (Example 4) compositions in accelerated conditions**

| **Stability testing at 40°C/75%RH** | | | |
|---|---|---|---|
| **Test parameter** | **Time point (months)** | **Examples** | |
| | | **Non-annealed** | **Annealed (example 4)** |
| **Assay** | | | |
| Assay (mg/mL) | 0 | 151.2 (99.8%) | 154.7 (106.1%) |
| | 1 | 155.4 (101.6%) | 155.4 (106.6%) |
| | 3 | 152.5 (101.7%) | 156.7 (107.5%) |
| | 6 | 153.3 (101.2%) | 155.3 (106.5%) |

| **Related Substances** | | | |
|---|---|---|---|
| Related substances-Total impurity (%) | 0 | 0.3 | < LOD |
| | 1 | 0.2 | < LOD |
| | 3 | 0.3 | < LOD |
| | 6 | 0.2 | 0.01 |

| **Dissolution** | | | |
|---|---|---|---|
| 2 min | 0 | 7 | 10 |
| 8 min | | 29 | 33 |
| 20 min | | 58 | 59 |
| 45 min | | 86 | 79 |
| 2 min | 1 | 6 | 8 |
| 8 min | | 23 | 29 |
| 20 min | | 47 | 54 |
| 45 min | | 73 | 75 |
| 2 min | 3 | 5 | 11 |
| 8 min | | 17 | 28 |
| 20 min | | 35 | 50 |
| 45 min | | 58 | 72 |
| 2 min | 6 | 5 | 8 |
| 8 min | | 13 | 25 |
| 20 min | | 30 | 47 |
| 45 min | | 53 | 68 |

| **Particle Size Distribution CPS** | | | |
|---|---|---|---|
| Surface mean (µm) | 0 | 1.78 | 0.44 |
| D10 (µm) | | 0.52 | 0.21 |
| D50 (µm) | | 1.73 | 0.38 |
| D90 (µm) | | 3.02 | 0.77 |
| Surface mean (µm) | 1 | 1.25 | 0.45 |
| D10 (µm) | | 0.37 | 0.21 |
| D50 (µm) | | 0.93 | 0.38 |
| D90 (µm) | | 2.57 | 0.78 |
| Surface mean (µm) | 3 | 1.15 | 0.49 |
| D10 (µm) | | 0.37 | 0.23 |
| D50 (µm) | | 0.80 | 0.42 |
| D90 (µm) | | 2.44 | 0.85 |
| Surface mean (µm) | 6 | 1.21 | 0.52 |
| D10 (µm) | | 0.38 | 0.24 |
| D50 (µm) | | 0.85 | 0.44 |
| D90 (µm) | | 2.52 | 0.91 |

| **Particle Size Distribution SALD** | | | |
|---|---|---|---|
| Volume mean (µm) | 0 | 8.09 | 1.03 |
| D10 (µm) | | 2.77 | 0.39 |
| D50 (µm) | | 9.04 | 1.06 |
| D90 (µm) | | 19.88 | 2.65 |
| Volume mean (µm) | 1 | 7.58 | 1.00 |
| D10 (µm) | | 2.62 | 0.40 |
| D50 (µm) | | 8.78 | 1.05 |
| D90 (µm) | | 19.94 | 2.25 |
| Volume mean (µm) | 3 | 8.23 | 1.11 |
| D10 (µm) | | 2.92 | 0.41 |
| D50 (µm) | | 9.23 | 1.17 |
| D90 (µm) | | 20.08 | 2.75 |
| Volume mean (µm) | 6 | 7.94 | 1.18 |
| D10 (µm) | | 2.78 | 0.42 |
| D50 (µm) | | 8.96 | 1.24 |
| D90 (µm) | | 19.71 | 2.94 |

**Table 10: Stability profile of non-annealed (Example 9) and annealed (Example 4) composition in standard conditions**

| **Stability testing at 25°C/60%RH** | | | |
|---|---|---|---|
| **Test parameter** | **Time point (months)** | **Examples** | |
| | | **Non-annealed** | **Annealed (Example 4)** |
| **Assay** | | | |
| Assay (mg/mL) | 0 | 151.2 (99.8%) | 154.7 (106.1%) |
| | 3 | 153.2 (99.2%) | 157.8 (108.3%) |
| | 6 | 152.4 (95.8%) | 154.5 (105.0%) |
| | 9 | 152.3 (NA) | 155.2 (106.4%) |
| | 12 | 154.1 (NA) | 154.5 (105.0%) |

| **Related Substances** | | | |
|---|---|---|---|
| Related substances- Total impurity (%) | 0 | 0.3 | < LOD |
| | 3 | 0.3 | < LOD |
| | 6 | 0.3 | < LOD |
| | 9 | 0.2 | < LOD |
| | 12 | 0.2 | < LOD |

| **Dissolution** | | | |
|---|---|---|---|
| 2 min | 0 | 7 | 10 |
| 8 min | | 29 | 33 |
| 20 min | | 58 | 59 |
| 45 min | | 86 | 79 |
| 2 min | 3 | 7 | 12 |
| 8 min | | 24 | 31 |
| 20 min | | 48 | 54 |
| 45 min | | 74 | 75 |
| 2 min | 6 | 6 | 9 |
| 8 min | | 25 | 30 |
| 20 min | | 49 | 55 |
| 45 min | | 73 | 75 |
| 2 min | 9 | 8 | 10 |
| 8 min | | 26 | 31 |
| 20 min | | 49 | 56 |
| 45 min | | 73 | 76 |
| 2 min | 12 | NA | 10 |
| 8 min | | | 31 |
| 20 min | | | 57 |
| 45 min | | | 78 |

| **Particle Size Distribution CPS (µm)** | | | |
|---|---|---|---|
| Surface mean (µm) | 0 | 1.78 | 0.44 |
| | | 0.52 | 0.21 |
| D10 (µm) | | 1.73 | 0.38 |
| D50 (µm) | | 3.02 | 0.77 |
| D90 (µm) | | | |
| Surface mean (µm) | 3 | 1.52 | 0.44 |
| D10 (µm) | | 0.40 | 0.21 |
| D50 (µm) | | 1.45 | 0.38 |
| D90 (µm) | | 2.79 | 0.77 |
| Surface mean (µm) | 6 | 1.51 | 0.43 |
| D10 (µm) | | 0.40 | 0.21 |
| D50 (µm) | | 1.44 | 0.37 |
| D90 (µm) | | 2.81 | 0.75 |
| Surface mean (µm) | 9 | NA | 0.45 |
| D10 (µm) | | | 0.21 |
| D50 (µm) | | | 0.38 |
| D90 (µm) | | | 0.77 |
| Surface mean (µm) | 12 | NA | 0.49 |
| D10 (µm) | | | 0.23 |
| D50 (µm) | | | 0.41 |
| D90 (µm) | | | 0.84 |

| **Particle Size Distribution SALD** | | | |
|---|---|---|---|
| Volume mean (µm) | 0 | 8.09 | 1.03 |
| D10 (µm) | | 2.77 | 0.39 |
| D50 (µm) | | 9.04 | 1.06 |
| D90 (µm) | | 19.88 | 2.65 |
| Volume mean (µm) | 3 | 8.00 | 1.01 |
| D10 (µm) | | 2.73 | 0.39 |
| D50 (µm) | | 8.90 | 1.05 |
| D90 (µm) | | 19.99 | 2.49 |
| Volume mean (µm) | 6 | 7.96 | 1.00 |
| D10 (µm) | | 2.74 | 0.39 |
| D50 (µm) | | 8.85 | 1.05 |
| D90 (µm) | | 19.84 | 2.38 |
| Volume mean (µm) | 9 | NA | NA |
| D10 (µm) | | NA | NA |
| D50 (µm) | | NA | NA |
| D90 (µm) | | NA | NA |
| Volume mean (µm) | 12 | NA | 1.07 |
| D10 (µm) | | NA | 0.44 |
| D50 (µm) | | NA | 1.09 |
| D90 (µm) | | NA | 2.57 |

**Table 11: Effect of annealing: impact of temperature on final PSD in CPS**

| Annealing Temperature (°C) | Annealing Duration (days) | CPS Analysis | | | |
|---|---|---|---|---|---|
| | | Surface mean (µm) | D10 (µm) | D50 (µm) | D90 (µm) |
| 40 | 16 | 0.46 | 0.21 | 0.39 | 0.73 |
| 60 | 2 | 0.58 | 0.25 | 0.50 | 0.98 |
| 80 | 2 | 1.57 | 0.82 | 1.39 | 2.64 |

**Table 12: Stability profile of annealed aripiprazole composition in accelerated conditions**

| **Stability testing at 40°C/75%RH** | | |
|---|---|---|
| **Test parameter** | **Time point (months)** | **Annealed (Example 8)** |
| **Assay** | | |
| Assay (mg/mL) | 0 | 209.2 |
| | 1 | 231.9 |
| | 3 | 210.3 |
| | 6 | 211.8 |

| **Related Substances** | | |
|---|---|---|
| Related substances-Total impurity (%) | 0 | < 0.05 |
| | 1 | < 0.05 |
| | 3 | < 0.05 |
| | 6 | < 0.05 |

| **Dissolution** | | |
|---|---|---|
| 15 min | 0 | 47 |
| 120 min | | 63 |
| 480 min | | 89 |
| 15 min | 1 | 45 |
| 120 min | | 63 |
| 480 min | | 87 |
| 15 min | 3 | 44 |
| 120 min | | 62 |
| 480 min | | 87 |
| 15 min | 6 | 43 |
| 120 min | | 62 |
| 480 min | | 87 |

| **Particle Size Distribution CPS** | | |
|---|---|---|
| Surface mean (µm) | 0 | 4.79 |
| D10 (µm) | | 1.78 |
| D50 (µm) | | 3.47 |
| D90 (µm) | | 9.25 |
| Surface mean (µm) | 1 | 5.76 |
| D10 (µm) | | 1.82 |
| D50 (µm) | | 3.74 |
| D90 (µm) | | 10.50 |
| Surface mean (µm) | 3 | 4.91 |
| D10 (µm) | | 1.85 |
| D50 (µm) | | 3.76 |
| D90 (µm) | | 9.77 |
| Surface mean (µm) | 6 | 4.85 |
| D10 (µm) | | 1.89 |
| D50 (µm) | | 3.80 |
| D90 (µm) | | 9.55 |

| **Particle Size Distribution SALD** | | |
|---|---|---|
| Volume mean (µm) | 0 | 7.62 |
| D10 (µm) | | 1.75 |
| D50 (µm) | | 8.40 |
| D90 (µm) | | 28.83 |
| Volume mean (µm) | 1 | 9.67 |
| D10 (µm) | | 2.28 |
| D50 (µm) | | 11.80 |
| D90 (µm) | | 32.97 |
| Volume mean (µm) | 3 | 8.58 |
| D10 (µm) | | 2.48 |
| D50 (µm) | | 10.02 |
| D90 (µm) | | 24.78 |
| Volume mean (µm) | 6 | 8.95 |
| D10 (µm) | | 2.71 |
| D50 (µm) | | 10.49 |
| D90 (µm) | | 24.77 |

**Table 13: Stability profile of annealed aripiprazole composition in standard conditions**

| **Stability testing at 25°C/60%RH** | | |
|---|---|---|
| **Test parameter** | **Time point (months)** | **Annealed (Example 8)** |
| **Assay** | | |
| Assay (mg/mL) | 0 | 209.2 |
| | 3 | 207.4 |
| | 6 | 202.9 |
| | 9 | 203.6 |
| | 12 | 207.1 |

| **Related Substances** | | |
|---|---|---|
| Related substances-Total impurity (%) | 0 | < 0.05 |
| | 3 | < 0.05 |
| | 6 | < 0.05 |
| | 9 | < 0.05 |
| | 12 | < 0.05 |

| **Dissolution** | | |
|---|---|---|
| 15 min | 0 | 47 |
| 120 min | | 63 |
| 480 min | | 89 |
| 15 min | 3 | 46 |
| 120 min | | 63 |
| 480 min | | 88 |
| 15 min | 6 | 46 |
| 120 min | | 65 |
| 480 min | | 89 |
| 15 min | 9 | 44 |
| 120 min | | 64 |
| 480 min | | 88 |
| 15 min | 12 | 44 |
| 120 min | | 62 |
| 480 min | | 86 |

| **Particle Size Distribution CPS** | | |
|---|---|---|
| Surface mean (µm) | 0 | 4.79 |
| D10 (µm) | | 1.78 |
| D50 (µm) | | 3.47 |
| D90 (µm) | | 9.25 |
| Surface mean (µm) | 3 | 5.00 |
| D10 (µm) | | 2.00 |
| D50 (µm) | | 3.83 |
| D90 (µm) | | 9.52 |
| Surface mean (µm) | 6 | 4.92 |
| D10 (µm) | | 1.98 |
| D50 (µm) | | 3.83 |
| D90 (µm) | | 9.38 |
| Surface mean (µm) | 9 | 4.67 |
| D10 (µm) | | 1.92 |
| D50 (µm) | | 3.70 |
| D90 (µm) | | 9.13 |
| Surface mean (µm) | 12 | 6.07 |
| D10 (µm) | | 2.21 |
| D50 (µm) | | 4.36 |
| D90 (µm) | | 10.70 |

| **Particle Size Distribution SALD** | | |
|---|---|---|
| Volume mean (µm) | 0 | 7.62 |
| D10 (µm) | | 1.75 |
| D50 (µm) | | 8.40 |
| D90 (µm) | | 28.83 |
| Volume mean (µm) | 3 | 9.47 |
| D10 (µm) | | 3.09 |
| D50 (µm) | | 11.05 |
| D90 (µm) | | 24.72 |
| Volume mean (µm) | 6 | 9.67 |
| D10 (µm) | | 3.00 |
| D50 (µm) | | 10.98 |
| D90 (µm) | | 26.68 |
| Volume mean (µm) | 9 | 9.45 |
| D10 (µm) | | 2.66 |
| D50 (µm) | | 10.91 |
| D90 (µm) | | 27.91 |
| Volume mean (µm) | 12 | 11.07 |
| D10 (µm) | | 3.09 |
| D50 (µm) | | 12.92 |
| D90 (µm) | | 33.69 |

**Table 14: Impact of the timing of the annealing step on PSD in CPS and SALD analysis**

| Timing of Annealing (t=60C) | Annealing Duration (hours) | CPS Analysis | | | |
|---|---|---|---|---|---|
| | | Surface mean (µm) | D10 (µm) | D50 (µm) | D90 (µm) |
| Before solvent removal | 48 | 5.09 | 0.80 | 4.07 | 10.67 |
| During solvent removal | 48 | 1.83 | 0.61 | 1.25 | 3.31 |
| | | | | | |
| Timing of Annealing | Annealing | SALD analysis | | | |

| (t=60C) | Duration (hours) | Volume mean (µm) | D10 (µm) | D50 (µm) | D90 (µm) |
|---|---|---|---|---|---|
| Before solvent removal | 48 | 13.1 | 3.2 | 15.2 | 47.0 |
| During solvent removal | 48 | 5.1 | 1.1 | 4.9 | 23.9 |

**Table 15: Stability of annealing at different timing in the process**

| **Stability testing at 40°C/75%RH** | | | |
|---|---|---|---|
| **Test parameter** | **Time point (months)** | **Examples** | |
| | | **Example 12a (Annealed before solvent removal)** | **Example 12b (Annealed during solvent removal)** |
| **Assay** | | | |
| Assay (mg/mL) | 0 | 287.7 | 287.5 |
| | 1 | 286.1 | 287.0 |
| | 3 | 289.3 | 286.3 |
| | 6 | 287.8 | 287.8 |

| **Related Substances** | | | |
|---|---|---|---|
| Related substances- Total impurity (%) | 0 | 0.12 | 0.05 |
| | 1 | 0.08 | <0.05 |
| | 3 | 0.08 | ND |
| | 6 | 0.08 | ND |

| **Dissolution** | | | |
|---|---|---|---|
| 30 min | 0 | 42.7 | 27.2 |
| 180 min | | 81.3 | 66.8 |
| 360 min | | 88.9 | 77.3 |
| 1140 min | | 96.4 | 92.9 |
| 30 min | 1 | 30.3 | 26.0 |
| 180 min | | 72.9 | 66.2 |
| 360 min | | 83.4 | 77.1 |
| 1140 min | | 94.0 | 92.2 |
| 30 min | 3 | 32.8 | 21.0 |
| 180 min | | 75.5 | 62.4 |
| 360 min | | 85.7 | 74.2 |
| 1140 min | | 96.6 | 91.2 |
| 30 min | 6 | 30.1 | 19.8 |
| 180 min | | 74.6 | 60.9 |
| 360 min | | 86.3 | 74.0 |
| 1140 min | | 98.3 | 92.3 |

| **Particle Size Distribution CPS** | | | |
|---|---|---|---|
| Surface mean (µm) | 0 | 1.10 | 1.83 |
| D10 (µm) | | 0.39 | 0.61 |
| D50 (µm) | | 0.74 | 1.25 |
| D90 (µm) | | 1.97 | 3.31 |
| Surface mean (µm) | 1 | 1.45 | 2.89 |
| D10 (µm) | | 0.42 | 0.65 |
| D50 (µm) | | 0.80 | 1.49 |
| D90 (µm) | | 2.59 | 6.85 |
| Surface mean (µm) | 3 | 1.82 | 2.01 |
| D10 (µm) | | 0.45 | 0.65 |
| D50 (µm) | | 0.87 | 1.36 |
| D90 (µm) | | 3.49 | 4.08 |
| Surface mean (µm) | 6 | 1.37 | 2.02 |
| D10 (µm) | | 0.49 | 0.68 |
| D50 (µm) | | 0.90 | 1.38 |
| D90 (µm) | | 2.79 | 4.26 |

| **Particle Size Distribution SALD** | | | |
|---|---|---|---|
| Volume mean (µm) | 0 | 2.88 | 5.14 |
| D10 (µm) | | 0.65 | 1.11 |
| D50 (µm) | | 3.41 | 4.88 |
| D90 (µm) | | 15.33 | 23.89 |
| Volume mean (µm) | 1 | 3.93 | 6.40 |
| D10 (µm) | | 0.80 | 1.31 |
| D50 (µm) | | 4.33 | 6.81 |
| D90 (µm) | | 16.63 | 28.44 |
| Volume mean (µm) | 3 | 4.30 | 6.34 |
| D10 (µm) | | 0.92 | 1.30 |
| D50 (µm) | | 4.87 | 6.84 |
| D90 (µm) | | 16.85 | 27.03 |
| Volume mean (µm) | 6 | 3.88 | 6.65 |
| D10 (µm) | | 0.86 | 1.29 |
| D50 (µm) | | 4.33 | 7.26 |
| D90 (µm) | | 15.29 | 30.61 |

## Claims

1. A process for preparing stable nanoparticles, wherein said nanoparticles are prepared by a process comprising:
a. dissolving active pharmaceutical ingredient in one or more organic solvent(s) optionally containing a surfactant;
b. adding the solution of step (a) to another solvent to produce a suspension of active pharmaceutical ingredient, wherein another solvent optionally contains a surfactant;
c. removing the organic solvent(s);
d. concentrating the resulting suspension of step (c);
e. further annealing the suspension either after step (b) or step (c) or step (d) at a temperature ranging from 30°C to 99°C for 0.5 hours to 2 weeks, at rate from 0.01°C/min to 50.0°C/min.
f. optionally, drying the nanoparticles of step (e).

2. The process of claim 1, wherein step e) comprises further annealing the suspension either after step (b) or step (c) or step (d) at a temperature ranging from 30°C to 40°C for 0.5 hours to 2 weeks, at rate from 0.01°C/min to 50.0°C/min.

3. The process of claim 1, wherein step e) comprises further annealing the suspension either after step (b) or step (c) or step (d) at a temperature ranging from 45°C to 65°C for 24 hours to 1 week, at rate from 0.1°C/min to 1.0°C/min.

4. The process of claim 1, wherein step e) comprises further annealing the suspension either after step (b) or step (c) or step (d) at a temperature ranging from 75°C to 95°C for 24 hours to 1 week at rate from 0.2°C/min to 0.8°C/min.

5. A pharmaceutical composition comprising nanoparticles obtained by the process of any one of claims 1-4.

6. The pharmaceutical composition according to claim 5, wherein the composition is selected from oral, parenteral, transdermal, rectal, urethral, intranasal, intra respiratory, intraocular, or conjunctival composition.

7. The pharmaceutical composition according to claim 5 or 6, wherein the composition comprises one or more pharmaceutically acceptable excipients.

8. A process for preparing stable nanoparticles with mean particle size less than about 30,000 nm, comprising the steps of:
a. preparing a solution by dissolving active pharmaceutical ingredient in one or more organic solvent(s);
b. adding the solution of step (a) to another solvent to produce a suspension of active pharmaceutical ingredient;
c. removing the organic solvent(s) from the suspension of step (b);
d. concentrating the resulting suspension of step (c);
e. further annealing the suspension either after step (b) or step (c) or step (d) at a temperature ranging from 30°C to 99°C for 0.5 hours to 2 weeks.

9. The process according to claim 8, wherein the organic solvent is selected from the group consisting of Tetrahydrofuran, Acetone, Dimethyl sulfoxide, Dimethylformamide, Dichloromethane, Acetonitrile, Triethanolamine and Acetic acid, Acetaldehyde, 1,2-Butanediol, 1,3-Butanediol, 1,4-Butanediol, 2-Butoxyethanol, Butyric Acid, Diethanolamine, Diethylenetriamine, Dimethoxyethane, 1,4-Dioxane, Ethylamine, Ethylene Glycol, Formic Acid, Furfuryl Alcohol, Glycerol, Methyl Diethanolamine, Methyl isocyanide, N-Methyl-2-pyrrolidone, 1,3-Propanediol, 1,5-Pentanediol, Propanoic Acid, Propylene Glycol, Pyridine, Triethylene glycol, Methyl t-butylether, Diethyl ether, Nitromethane, Diethtylene Glycol, 2-Butanone, Methyl Acetate, Ethyl Acetate, Benzyl alcohol, water or mixture of water, alcohols like methanol, ethanol, propanol, butanol and combinations thereof.

10. The process according to claim 8 or claim 9, wherein the another solvent is selected from the group consisting of water, water for injection, acetone, alcohols like methanol, ethanol, propanol, butanol and combinations thereof.

11. The process according to any one of claims 8-10, wherein the organic solvent is removed using a method selected from purging with gas, distillation, extraction or supercritical fluid extraction.

12. The process according to any one of claims 8-11, wherein the suspension is concentrated using a method selected from tangential flow filtration, centrifugation/sedimentation or dead-end filtration.

13. The process according to any one of claims 8-12, wherein the annealing step is carried at heating and cooling rate from 0.01°C/min to 50.0°C/min.

14. The process according to one of claims 8-13, wherein the organic solvent and another solvent optionally contains a surfactant.

15. The process according to claim 14, wherein the surfactant is selected from the group consisting of cetyl pyridinium chloride, gelatin, casein, lecithin (phosphatides), dextran, glycerol, gum acacia, cholesterol, tragacanth, stearic acid, benzalkonium chloride, carrageenan, diethanolamine, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters (Polysorbates); ethylene glycol, glycol esters, ethylene glycol esters, polyethylene glycols dodecyl trimethyl ammonium bromide, aliphatic alcohols, lanolin and alcohols thereof, fatty acids, mineral oils, polyoxyethylene stearates, colloidal silicon dioxide, phosphates, sodium dodecyl sulfate, carboxymethylcellulose calcium, hydroxypropyl celluloses, methylcellulose, hydroxyethylcellulose, hydroxypropylmethylcellulose, hydroxypropylmethyl-cellulose phthalate, non-crystalline cellulose, magnesium aluminum silicate, triethanolamine, polyvinyl alcohol, 4-(1,1,3,3-tetramethylbutyl)-phenol polymer with ethylene oxide and formaldehyde (also known as tyloxapol, superione, and triton), poloxamers; poloxamines; a charged phospholipid such as dimyristoyl phosphatidyl glycerol, dioctylsulfosuccinate (DOSS), dialkylesters of sodium sulfosuccinic acid sodium lauryl sulfate; alkyl aryl polyether; p-isononylphenoxypoly-(glycidol),; decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-nonyl β-D-glucopyranoside; octanoyl-N-methylglucamide; n-octyl-β-D-glucopyranoside; octyl β-D-thioglucopyranoside and the like or combinations thereof.

16. The composition comprising nanoparticles prepared according to any one of claims 8-15, wherein the nanoparticles are formulated in dosage form selected from including oral, parenteral, transdermal, rectal, urethral, intranasal, intra respiratory, intraocular, intravenous, intraarterial, conjunctival or any other form of local or systemic delivery

17. The composition according to claim 16, wherein the dosage form is parenteral preparation.

18. The composition according to claim 16 or claim 17, wherein parenteral preparation further comprises one or more suitable pharmaceutically acceptable excipient(s).

19. The composition according to claim 18, wherein the one or more pharmaceutically acceptable excipients are selected from the group consisting of viscosity enhancing agent(s), bulking agent(s), tonicity adjusting agent(s), wetting agent(s), surfactant(s), buffering agent(s), pH adjusting agent(s) and vehicle(s).

## Patentansprüche

1. Verfahren zur Herstellung stabiler Nanopartikel, wobei die Nanopartikel durch ein Verfahren hergestellt werden, umfassend:
a. Auflösen eines pharmazeutischen Wirkstoffs in einem oder mehreren organischen Lösungsmittel(n), das/die optional ein Tensid enthält/enthalten;
b. Zugeben der Lösung aus Schritt (a) zu einem anderen Lösungsmittel, um eine Suspension eines pharmazeutischen Wirkstoffs herzustellen, wobei ein anderes Lösungsmittel optional ein Tensid enthält;
c. Entfernen des/der organischen Lösungsmittel(s);
d. Konzentrieren der resultierenden Suspension aus Schritt (c);
e. weiteres Tempern der Suspension entweder nach Schritt (b) oder Schritt (c) oder Schritt (d) bei einer Temperatur im Bereich von 30 °C bis 99 °C für 0,5 Stunden bis 2 Wochen mit einer Rate von 0,01 °C/min bis 50,0 °C/min.
f. optionales Trocknen der Nanopartikel aus Schritt (e).

2. Verfahren nach Anspruch 1, wobei Schritt e) weiteres Tempern der Suspension entweder nach Schritt (b) oder Schritt (c) oder Schritt (d) bei einer Temperatur im Bereich von 30 °C bis 40 °C für 0,5 Stunden bis 2 Wochen mit einer Rate von 0,01 °C/min bis 50,0 °C/min umfasst.

3. Verfahren nach Anspruch 1, wobei Schritt e) weiteres Tempern der Suspension entweder nach Schritt (b) oder Schritt (c) oder Schritt (d) bei einer Temperatur im Bereich von 45 °C bis 65 °C für 24 Stunden bis 1 Woche mit einer Rate von 0,1 °C/min bis 1,0 °C/min umfasst.

4. Verfahren nach Anspruch 1, wobei Schritt e) weiteres Tempern der Suspension entweder nach Schritt (b) oder Schritt (c) oder Schritt (d) bei einer Temperatur im Bereich von 75 °C bis 95 °C für 24 Stunden bis 1 Woche mit einer Rate von 0,2 °C/min bis 0,8 °C/min umfasst.

5. Pharmazeutische Zusammensetzung, umfassend Nanopartikel, die durch das Verfahren nach einem der Ansprüche 1-4 erhalten werden.

6. Pharmazeutische Zusammensetzung nach Anspruch 5, wobei die Zusammensetzung ausgewählt ist aus oraler, parenteraler, transdermaler, rektaler, urethraler, intranasaler, intrarespiratorischer, intraokularer oder konjunktivaler Zusammensetzung.

7. Pharmazeutische Zusammensetzung nach Anspruch 5 oder 6, wobei die Zusammensetzung einen oder mehrere pharmazeutisch akzeptable Hilfsstoffe umfasst.

8. Verfahren zur Herstellung stabiler Nanopartikel mit einer mittleren Partikelgröße von weniger als etwa 30.000 nm, umfassend die Schritte:
a. Herstellen einer Lösung durch Auflösen eines pharmazeutischen Wirkstoffs in einem oder mehreren organischen Lösungsmittel(n);
b. Zugeben der Lösung aus Schritt (a) zu einem anderen Lösungsmittel, um eine Suspension eines pharmazeutischen Wirkstoffs herzustellen;
c. Entfernen des/der organischen Lösungsmittel(s) aus der Suspension aus Schritt (b);
d. Konzentrieren der resultierenden Suspension aus Schritt (c);
e. weiteres Tempern der Suspension entweder nach Schritt (b) oder Schritt (c) oder Schritt (d) bei einer Temperatur im Bereich von 30 °C bis 99 °C für 0,5 Stunden bis 2 Wochen.

9. Verfahren nach Anspruch 8, wobei das organische Lösungsmittel ausgewählt ist aus der Gruppe bestehend aus Tetrahydrofuran, Aceton, Dimethylsulfoxid, Dimethylformamid, Dichlormethan, Acetonitril, Triethanolamin und Essigsäure, Acetaldehyd, 1,2-Butandiol, 1,3-Butandiol, 1,4-Butandiol, 2-Butoxyethanol, Buttersäure, Diethanolamin, Diethylentriamin, Dimethoxyethan, 1,4-Dioxan, Ethylamin, Ethylenglycol, Ameisensäure, Furfurylalkohol, Glycerin, Methyldiethanolamin, Methylisocyanid, N-Methyl-2-pyrrolidon, 1,3-Propandiol, 1,5-Pentandiol, Propansäure, Propylenglycol, Pyridin, Triethylenglycol, Methyl-t-butylether, Diethylether, Nitromethan, Diethtylenglycol, 2-Butanon, Methylacetat, Ethylacetat, Benzylalkohol, Wasser oder einer Mischung aus Wasser, Alkoholen wie Methanol, Ethanol, Propanol, Butanol und Kombinationen davon.

10. Verfahren nach Anspruch 8 oder Anspruch 9, wobei das andere Lösungsmittel ausgewählt ist aus der Gruppe bestehend aus Wasser, Wasser zur Injektion, Aceton, Alkoholen wie Methanol, Ethanol, Propanol, Butanol und Kombinationen davon.

11. Verfahren nach einem der Ansprüche 8-10, wobei das organische Lösungsmittel unter Verwendung eines Verfahrens entfernt wird, das ausgewählt ist aus Spülen mit Gas, Destillation, Extraktion oder Extraktion mit überkritischem Fluid.

12. Verfahren nach einem der Ansprüche 8-11, wobei die Suspension unter Verwendung eines Verfahrens konzentriert wird, das ausgewählt ist aus Tangentialflussfiltration, Zentrifugation/Sedimentation oder Dead-End-Filtration.

13. Verfahren nach einem der Ansprüche 8-12, wobei der Temperschritt mit einer Heiz- und Kühlrate von 0,01 °C/min bis 50,0 °C/min durchgeführt wird.

14. Verfahren nach einem der Ansprüche 8-13, wobei das organische Lösungsmittel und ein anderes Lösungsmittel optional ein Tensid enthält.

15. Verfahren nach Anspruch 14, wobei das Tensid ausgewählt ist aus der Gruppe bestehend aus Cetylpyridiniumchlorid, Gelatine, Casein, Lecithin (Phosphatiden), Dextran, Glycerin, Akaziengummi, Cholesterin, Tragant, Stearinsäure, Benzalkoniumchlorid, Carrageenan, Diethanolamin, Calciumstearat, Glycerinmonostearat, Cetostearylalkohol, Cetomacrogol-Emulgierwachs, Sorbitanestern, Polyoxyethylenalkylethern, Polyoxyethylenrizinusölderivaten, Polyoxyethylensorbitanfettsäureestern (Polysorbaten); Ethylenglycol, Glycolestern, Ethylenglycolestern, Polyethylenglycolen, Dodecyltrimethylammoniumbromid, aliphatischen Alkoholen, Lanolin und Alkoholen davon, Fettsäuren, Mineralölen, Polyoxyethylenstearaten, kolloidalem Siliciumdioxid, Phosphaten, Natriumdodecylsulfat, Carboxymethylcellulose-Calcium, Hydroxypropylcellulosen, Methylcellulose, Hydroxyethylcellulose, Hydroxypropylmethylcellulose, Hydroxypropylmethylcellulosephthalat, nichtkristalliner Cellulose, Magnesiumaluminiumsilicat, Triethanolamin, Polyvinylalkohol, 4-(1,1,3,3-Tetramethylbutyl)-phenolpolymer mit Ethylenoxid und Formaldehyd (auch bekannt als Tyloxapol, Superion und Triton), Poloxameren; Poloxaminen; einem geladenen Phospholipid wie Dimyristoylphosphatidylglycerin, Dioctylsulfosuccinat (DOSS), Dialkylestern von Natriumsulfobernsteinsäure-Natriumlaurylsulfat; Alkylarylpolyether; p-Isononylphenoxypoly-(glycidol), Decanoyl-N-methylglucamid; n-Decyl-β-D-glucopyranosid; n-Decyl-β-D-maltopyranosid; n-Dodecyl-β-D-glucopyranosid; n-Dodecyl-β-D-maltosid; Heptanoyl-N-methylglucamid; n-Heptyl-β-D-glucopyranosid; n-Heptyl-β-D-thioglucosid; n-Hexyl-β-D-glucopyranosid; Nonanoyl-N-methylglucamid; n-Nonyl-β-D-glucopyranosid; Octanoyl-N-methylglucamid; n-Octyl-β-D-glucopyranosid; Octyl-β-D-thioglucopyranosid und dergleichen oder Kombinationen davon.

16. Zusammensetzung, umfassend Nanopartikel, hergestellt nach einem der Ansprüche 8-15, wobei die Nanopartikel in einer Dosierungsform formuliert sind, die ausgewählt ist aus einschließlich oraler, parenteraler, transdermaler, rektaler, urethraler, intranasaler, intrarespiratorischer, intraokularer, intravenöser, intraarterieller, konjunktivaler oder jeder anderen Form von lokaler oder systemischer Verabreichung.

17. Zusammensetzung nach Anspruch 16, wobei die Dosierungsform eine parenterale Zubereitung ist.

18. Zusammensetzung nach Anspruch 16 oder Anspruch 17, wobei die parenterale Zubereitung ferner einen oder mehrere geeignete pharmazeutisch akzeptable Hilfsstoffe umfasst.

19. Zusammensetzung nach Anspruch 18, wobei der eine oder die mehreren pharmazeutisch akzeptablen Hilfsstoffe ausgewählt sind aus der Gruppe bestehend aus viskositätssteigernden Mittel(n), Füllstoff(en), Tonizitätseinstellmittel(n), Benetzungsmittel(n), Tensid(en), Puffermittel(n), pH-Einstellmittel(n) und Vehikel(n).

## Revendications

1. Procédé de préparation de nanoparticules stables, dans lequel lesdites nanoparticules sont préparées par un procédé comprenant :
a. la dissolution d'ingrédient pharmaceutique actif dans un ou plusieurs solvants organiques contenant éventuellement un tensioactif ;
b. l'ajout de la solution de l'étape (a) à un autre solvant pour produire une suspension d'ingrédient pharmaceutique actif, où l'autre solvant contient éventuellement un tensioactif ;
c. l'élimination du ou des solvants organiques ;
d. la concentration de la suspension résultante de l'étape (c) ;
e. le recuit ultérieur de la suspension soit après l'étape (b) soit après l'étape (c) soit après l'étape (d) à une température allant de 30°C à 99°C pendant 0,5 heure à 2 semaines, à un taux de 0,01°C/min à 50,0°C/min ;
f. éventuellement, le séchage des nanoparticules de l'étape (e).

2. Procédé selon la revendication 1, dans lequel l'étape (e) comprend le recuit ultérieur de la suspension soit après l'étape (b) soit après l'étape (c) soit après l'étape (d) à une température allant de 30°C à 40°C pendant 0,5 heure à 2 semaines, à un taux de 0,01°C/min à 50,0°C/min.

3. Procédé selon la revendication 1, dans lequel l'étape (e) comprend le recuit ultérieur de la suspension soit après l'étape (b) soit après l'étape (c) soit après l'étape (d) à une température allant de 45°C à 65°C pendant 24 heures à 1 semaine, à un taux de 0,1°C/min à 1,0°C/min.

4. Procédé selon la revendication 1, dans lequel l'étape (e) comprend le recuit ultérieur de la suspension soit après l'étape (b) soit après l'étape (c) soit après l'étape (d) à une température allant de 75°C à 95°C pendant 24 heures à 1 semaine, à un taux de 0,2°C/min à 0,8°C/min.

5. Composition pharmaceutique comprenant des nanoparticules obtenues par le procédé selon l'une quelconque des revendications 1 à 4.

6. Composition pharmaceutique selon la revendication 5, la composition étant choisie parmi une composition orale, parentérale, transdermique, rectale, uréthrale, intranasale, intrarespiratoire, intraoculaire ou conjonctivale.

7. Composition pharmaceutique selon la revendication 5 ou 6, la composition comprenant un ou plusieurs excipients pharmaceutiquement acceptables.

8. Procédé de préparation de nanoparticules stables ayant une taille moyenne de particule inférieure à environ 30 000 nm, comprenant les étape de :
a. préparation d'une solution par dissolution d'un ingrédient pharmaceutique actif dans un ou plusieurs solvants organiques ;
b. ajout de la solution de l'étape (a) à un autre solvant pour produire une suspension d'ingrédient pharmaceutique actif ;
c. élimination du ou des solvants organiques de la suspension de l'étape (b) ;
d. concentration de la suspension résultante de l'étape (c) ;
e. recuit ultérieur de la suspension soit après l'étape (b) soit après l'étape (c) soit après l'étape (d) à une température allant de 30°C à 99°C pendant 0,5 heure à 2 semaines.

9. Procédé selon la revendication 8, dans lequel le solvant organique est choisi dans le groupe consistant en tétrahydrofurane, acétone, diméthylsulfoxyde, diméthylformamide, dichlorométhane, acétonitrile, triéthanolamine et acide acétique, acétaldéhyde, 1,2-butanediol, 1,3-butanediol, 1,4-butanediol, 2-butoxyéthanol, acide butyrique, diéthanolamine, diéthylènetriamine, diméthoxyéthane, 1,4-dioxane, éthylamine, éthylène glycol, acide formique, alcool furfurylique, glycérol, méthyldiéthanolamine, isocyanure de méthyle, N-méthyl-2-pyrrolidone, 1,3-propanediol, 1,5-pentanediol, acide propanoïque, propylène glycol, pyridine, triéthylène glycol, méthyl t-butyl éther, diéthyl éther, nitrométhane, diéthylène glycol, 2-butanone, acétate de méthyle, acétate d'éthyle, alcool benzylique, eau ou mélange d'eau, d'alcools comme le méthanol, l'éthanol, le propanol, le butanol et des combinaisons de ceux-ci.

10. Procédé selon la revendication 8 ou la revendication 9, dans lequel l'autre solvant est choisi dans le groupe consistant en eau, eau pour injection, acétone, alcools comme le méthanol, l'éthanol, le propanol, le butanol et des combinaisons de ceux-ci.

11. Procédé selon l'une quelconque des revendications 8 à 10, dans lequel le solvant organique est éliminé en utilisant une méthode choisie parmi la purge avec du gaz, la distillation, l'extraction ou l'extraction par fluide supercritique.

12. Procédé selon l'une quelconque des revendications 8 à 11, dans lequel la suspension est concentrée en utilisant une méthode choisie parmi la filtration tangentielle, la centrifugation/sédimentation ou la filtration frontale.

13. Procédé selon l'une quelconque des revendications 8 à 12, dans lequel l'étape de recuit est réalisée à un taux de chauffe et de refroidissement allant de 0,01°C/min à 50,0°C/min.

14. Procédé selon l'une quelconque des revendications 8 à 13, dans lequel le solvant organique et l'autre solvant contiennent éventuellement un tensioactif.

15. Procédé selon la revendication 14, dans lequel le tensioactif est choisi dans le groupe consistant en chlorure de cétylpyridinium, gélatine, caséine, lécithine (phosphatides), dextrane, glycérol, gomme acacia, cholestérol, tragacanthe, acide stéarique, chlorure de benzalconium, carraghénane, diéthanolamine, stéarate de calcium, monostéarate de glycérol, alcool cétostéarylique, cire émulsifiante au cétomacrogol, esters de sorbitane, éthers alkyliques de polyoxyéthylène, dérivés d'huile de ricin polyoxyéthylénés, esters d'acide gras de polyoxyéthylène sorbitane (polysorbates) ; éthylène glycol, esters de glycol, esters d'éthylène glycol, polyéthylène glycols, bromure de dodécyl triméthyl ammonium, alcools aliphatiques, lanoline et alcools de celle-ci, acides gras, huiles minérales, stéarates de polyoxyéthylène, dioxyde de silicium colloïdal, phosphates, dodécylsulfate de sodium, carboxyméthylcellulose calcique, hydroxypropyl celluloses, méthylcellulose, hydroxyéthylcellulose, hydroxypropyl méthylcellulose, phtalate d'hydroxypropylméthylcellulose, cellulose non cristalline, silicate de magnésium aluminium, triéthanolamine, alcool polyvinylique, polymère de 4-(1,1,3,3-tétraméthylbutyl)-phénol avec de l'oxyde d'éthylène et du formaldéhyde (également connu sous le nom de tyloxapol, supérione et triton), poloxamères ; poloxamines ; un phospholipide chargé tel que le dimyristoyl phosphatidylglycérol, le dioctylsulfosuccinate (DOSS), les esters dialkyliques de laurylsulfate de sodium d'acide sulfosuccinique sodique ; alkyl aryl polyéther ; p-isononylphénoxypoly-(glycidol) ; décanoyl-N-méthylglucamide ; n-décyl β-D-glucopyranoside ; n-décyl β-D-maltopyranoside ; n-dodécyl β-D-glucopyranoside ; n-dodécyl β-D-maltoside ; heptanoyl-N-méthylglucamide ; n-heptyl-β-D-glucopyranoside ; n-heptyl β-D-thioglucoside ; n-hexyl β-D-glucopyranoside ; nonanoyl-N-méthylglucamide ; n-nonyl β-D-glucopyranoside ; octanoyl-N-méthylglucamide ; n-octyl-β-D-glucopyranoside ; octyl β-D-thioglucopyranoside et similaire ou des combinaisons de ceux-ci.

16. Composition comprenant des nanoparticules préparées selon l'une quelconque des revendications 8 à 15, dans laquelle les nanoparticules sont formulées en une forme de dosage choisie parmi orale, parentérale, transdermique, rectale, uréthrale, intranasale, intrarespiratoire, intraoculaire, intraveineuse, intra-artérielle, conjonctivale ou toute autre forme d'administration locale ou systémique.

17. Composition selon la revendication 16, dans laquelle la forme de dosage est une préparation parentérale.

18. Composition selon la revendication 16 ou la revendication 17, dans laquelle la préparation parentérale comprend en outre un ou plusieurs excipients pharmaceutiquement acceptables appropriés.

19. Composition selon la revendication 18, dans laquelle les un ou plusieurs excipients pharmaceutiquement acceptables sont choisis dans le groupe consistant en agent(s) épaississant(s), agent(s) de charge, agent(s) régulateur(s) de tonicité, agent(s) mouillant(s), tensioactif(s), agent(s) tampon(s), agent(s) régulateur(s) de pH et véhicule(s).
